# EUROPEAN PATENT APPLICATION

(11) **EP 2 535 413 A2**
(43) Date of publication of application: **19.12.2012**
(21) Application number: 12176988.9
(22) Date of filing: 10.11.2006
(51) Int. Cl.: C12N 15/113, A61K 31/712, A61K 31/7125, C07H 21/00, A61P 1/16, A61P 29/00, A61P 31/00

(54) **Splice switching oligomers for the TNF superfamily receptors and their use in treatment of disease**

(30) Priority: 10.11.2005 US 735429 P; 20.10.2006 US 862350 P
(62) Divisional of application: 06827673.2
(71) Applicant: Santaris Pharma A/S, 2970 Hørsholm (DK); The University of North Carolina at Chapel Hill, Chapel Hill (US); Ercole Biotech, INC., NC 27709 (US)
(72) Inventor: Sazani, Peter L., Chapel Hill, NC 27514 (US); Kole, Ryszard, Chapel Hill, NC 27514 (US); Ørum, Henrik, 3500 Værløse (DK)
(74) Representative: Inspicos A/S

(57) **Abstract**

Methods and compositions are disclosed for controlling expression of TNF receptors (TNFR1 and TNFR2) and of other receptors in the TNFR superfamily using compounds that modulate splicing of pre-mRNA encoding these receptors. More specifically these compounds cause the removal of the transmembrane domains of these receptors and produce soluble forms of the receptor which act as an antagonist to reduce TNF-α activity or activity of the relevant ligand. Reducing TNF- α activity provides a method of treating or ameliorating inflammatory diseases or conditions associated with TNF-α activity. Similarly, diseases associated with other ligands can be treated in like manner. In particular, the compounds of the invention are splice-splice switching oligomers (SSOs) which are small molecules that are stable *in vivo,* hybridize to the RNA in a sequence specific manner and, in conjunction with their target, are not degraded by RNAse H.

## Description

This application claims priority to U.S. Provisional application Ser. No. 60/862,350, filed October 20, 2006 and U.S. Provisional application Ser. No. 60/735,429, filed November 10, 2005 which are incorporated by reference herein in their entirety.

### FIELD OF THE INVENTION

The present invention relates to compositions and methods for controlling splicing of pre-mRNA molecules and regulating protein expression with splice switching oligonucleotides or splice switching oligomers (SSOs). SSOs are not limited to nucleotides but include any polymer or molecule that is able to hybridize to a target RNA with sequence specificity and does not activate RNase H or otherwise lead to degradation of the target RNA. Specifically described embodiments concern receptors for the tumor necrosis factor (TNF) superfamily.

### BACKGROUND OF THE INVENTION

The production of mRNA by eukaryotic cells is a two-stage process. First, a long contiguous transcript, pre-messenger RNA (pre-mRNA), is formed. The pre-mRNA contains sequences that code for protein (exons) interspersed with sequences that do not code for protein (introns). Second, the introns of the transcript are removed and the exons are joined by a process called splicing. This process is a key step in generation of mature, functional mRNA. The 5' end of each intron contains a splice-donor site or 5' splice site, and the 3' end of each intron contains a splice acceptor or 3' splice site. Processing of pre-mRNA involves a complex containing protein and RNA molecules, referred to collectively as the spliceosome, which carries out splicing and transport of mRNA from the nucleus.

When alternative splice sites are present, the splicing step permits the synthesis of two or more (related) proteins from a single gene (*See, e.g.,* Gist, A., 2005, Scientific American, April, p.60). Among the genes that employ alternative splicing as a physiological mechanism are the cell-surface receptors for protein cytokines that influence the inflammatory and immune system. These proteins are expressed in an integral membrane form and transduce signals in response to cytokine ligand binding. Such cytokine receptors also exist as a secreted form that can bind cytokine and prevent signal transduction. These two receptor forms are produced by alternative splicing and differ by the deletion of the one or more exons needed to encode the membrane-spanning domain of the molecule. For some receptors, a soluble fragment of the receptors, distinct from the secreted splice variants, is produced by proteolytic cleavage of the extracellular domain from the integral membrane bound receptors.

One such family of receptors is the TNF receptor (TNFR) superfamily. The TNFR superfamily currently consists of 29 receptors that mediate cellular signaling as a consequence of binding to one or more of the 19 ligands currently identified in the TNF superfamily. The TNFR superfamily is a group of type I transmembrane proteins, with a carboxy-terminal intracellular domain and an amino-terminal extracellular domain characterized by a common cysteine rich domain (CRD). The TNFR superfamily can be divided into two subgroups: receptors containing the intracellular death domain (DD) and those lacking it. The DD is an 80 amino acid motif that is responsible for the induction of apoptosis following receptor activation. Additionally, TNF-α receptor type I (TNFSFR1A, hereafter "TNFR1", exemplified by GenBank accession number X55313 for human mRNA) and TNF-α receptor type II (TNFSF1B, hereafter "TNFR2", exemplified by GenBank accession number NM_001066 for human mRNA) have a unique domain in common, called the pre-ligand-binding assembly domain (PLAD) that is required for assembly of multiple receptor subunits and subsequent binding to TNF-α. Most members of the TNFR superfamily activate signal transduction by associating with TNFR-associated factors (TRAFs). The association is mediated by specific motifs in the intracellular domain of TNFR superfamily members. (Palladino, M.A., et al., 2003, Nat. Rev. Drug Discov. 2:736-46). Other members of the TNFR superfamily include RANK (TNFRSF11A), CD40 (TNFRSF5), CD30 (TNFRSF8), and LT-βR (TNFRSF3).

TNF-α is a pro-inflammatory cytokine that exists as a membrane-bound homotrimer and is released into the circulation by the protease TNF-α converting enzyme (TACE). TNF-α is introduced into the circulation as a mediator of the inflammatory response to injury and infection. TNF-α activity is implicated in the progression of inflammatory diseases such as rheumatoid arthritis, Crohn's disease, ulcerative colitis, psoriasis and psoriatic arthritis (Palladino, M.A., et al., 2003, Nat. Rev. Drug Discov. 2:736-46). The acute exposure to high levels of TNF-α, as experienced during a massive infection, results in sepsis; its symptoms include shock, hypoxia, multiple organ failure, and death. Chronic low doses of TNF-α can cause cachexia, a disease characterized by weight loss, dehydration and fat loss, and is associated with malignancies.

TNF-α activity is mediated primarily through two receptors coded by two different genes, TNFR1 and TNFR2. TNFR1 is a membrane-bound protein with a molecular weight of approximately 55 kilodaltons (kDal), while TNFR2 is a membrane-bound protein with a molecular weight of 75 kDal. The soluble extracellular domains of both receptors are shed to some extent from the cell membrane by the action of metalloproteases. Moreover, the pre-mRNA of TNFR2 undergoes alternative splicing, creating either a full length, active membrane-bound receptor (mTNFR2), or a secreted decoy receptor (sTNFR2) that lacks exons 7 and 8 which encompasses the coding sequences for the transmembrane (Lainez et al., 2004, Int. Immunol., 16;169). The sTNFR2 binds TNF-α but does not elicit a physiological response, thus reducing TNF-α activity. Although an endogenous, secreted splice variant of TNFR1 has not yet been identified, the similar gene structures of the two receptors strongly suggest the potential to produce this TNFR1 isoform.

Knockout mice lacking both TNFR1 and TNFR2 treated with drugs that target the TNF signaling pathways indicate such drugs may be beneficial in treating stroke or traumatic brain injury (Bruce, et al., 1996, Nat. Med. 2:788). TNFR2 knockout mice were also used to establish a role for TNFR2 in experimentally-induced cerebral malaria (Lucas, R., et al., 1997, Eur. J. Immunol. 27:1719) and autoimmune encephalomyelitis (Suvannavejh, G.C., et al., 2000, Cell. Immunol., 205:24), models for human cerebral malaria and multiple sclerosis, respectively.

TNFR2 is present at high density on T cells and appears to play a role in the immune responses that lead to alveolitis in the pulmonary microenvironment of interstitial lung disease (Agostini, C., et al., 1996, Am. J. Respir. Crit. Care Med., 153:1359). TNFR2 is also implicated in human metabolic disorders of lipid metabolism and has been associated with obesity and insulin resistance (Fernandez-Real, et al., 2000, Diabetes Care, 23:831), familial combined hyperlipidemia (Geurts, et al., 2000, Hum. Mol. Genet. 9:2067; van Greevenbroek, et al., 2000, Atherosclerosis, 153:1), hypertension and hypercholesterolemia (Glenn, et al., 2000, Hum. Mol. Genet., 9:1943). TNFR2 has recently been associated with human narcolepsy (Komata, T., et al., 1999, Tissue Antigens, 53:527). In addition, TNFR2 polymorphism appears to lead to susceptibility to systemic lupus erythematosus (Hohjoh, H., et al., 2000, Tissue Antigens, 56:446).

Splice variants of CD40 (Tone, M., et al., 2001, Proc. Natl. Acad. Sci. 98:1751) ("Tone"), and CD95 (FAS) (Shen, L., et al., 2002, Am. J. Path. 161:2123), have been found in malignancies. Several of these splice variants result in loss of the transmembrane region due to deletion or due to mutations affecting the reading frame of exon 7. Whether these represent aberrant variants resulting from malignant transformation or physiological alternatives is not yet known.

Because of the role played by excessive activity by TNF superfamily members, it would be useful to control the alternative splicing of TNFR receptors so that the amount of the secreted form is increased and the amount of the integral membrane form is decreased. The present invention provides splice switching oligonucleotides or splice switching oligomers (SSOs) to achieve this goal. SSOs are similar to antisense oligonucleotides (ASONs). However, in contrast to ASON, SSOs are able to hybridize to a target RNA without causing degradation of the target by RNase H

SSOs have been used to modify the aberrant splicing found in certain thalassemias (U.S. Pat. No. 5,976,879 to Kole; Lacerra, G., et al., 2000, Proc. Natl. Acad. Sci. 97:9591). Studies with the IL-5 receptor α-chain (IL-5Rα) demonstrated that SSOs directed against the membrane-spanning exon increased synthesis of the secreted form and inhibited synthesis of the integral membrane form (U.S. Pat. No. 6,210,892 to Bennett; Karras, J.G., et al., 2000, Mol. Pharm, 58:380).

The IL-5 receptor is a member of a receptor type that occurs as a heterodimer. The interleukin 5 receptor (IL-5R) is a member of the IL-3R family of receptors, which also includes interleukin 3 receptor (IL-3R) and GM-CSF. IL-3R family members are multisubunit receptors consisting of a shared common β chain, and a unique α chain that conveys cytokine ligand specificity. IL-3R family members are expressed in the hematopoietic system. In particular, IL-5 is expressed exclusively in eosinophils, basophils and B cells (Adachiand, T. & Alam, R., 1998, Am. J. Physiol. 275:C623-33). These receptors and the TNFR superfamily of the present invention have no sequence homology and operate in distinct signaling pathways.

SSOs have been used to produce the major CD40 splice variant detected in Tone, in which deletion of exon 6, which is upstream of the transmembrane region, resulted in an altered reading frame of the protein. While the SSO resulted in the expected mRNA splice variant, the translation product of the variant mRNA appeared to be unstable because the secreted receptor could not be detected (Siwkowski, A.M., et al., 2004, Nucleic Acids Res. 32; 2695).

### SUMMARY OF THE INVENTION

The present invention provides compositions and methods for controlling expression of TNF receptors (TNFR1 and TNFR2) and of other cytokine receptors from the TNFR superfamily by controlling the splicing of pre-mRNA that codes for the said receptors. More specifically, the invention causes the increased expression of the secreted form and the decreased expression of the integral-membrane form. Furthermore, the invention can be used in the treatment of diseases associated with excessive cytokine activity.

The exon or exons that are present in the integral membrane form mRNA but are removed from the primary transcript (the "pre-mRNA") to make a secreted form mRNA are termed the "transmembrane exons." The invention involves nucleic acids and nucleic acid analogs that are complementary to either of the transmembrane exons and/or adjacent introns of a receptor pre-mRNA. Complementarity can be based on sequences in the sequence of pre-mRNA that spans the splice site, which would include, but is not limited to, complemtarity based on sequences that span the exon-intron junction, or complementarity can be based solely on the sequence of the intron, or complementarity can be based solely on the sequence of the exon.

There are several alternative chemistries available and known to those skilled in the art. One important feature is the ability to hybridize to a target RNA without causing degradation of the target by RNase H as do 2'-deoxy oligonucleotides ("antisense oligonucleotides" hereafter "ASON"), For clarity, such compounds will be termed splice-switching oligomers (SSOs). Those skilled in the art appreciate that SSO include, but are not limited to, 2' O-modified oligonucleotides and ribonucleosidephosphorothioates as well as peptide nucleic acids and other polymers lacking ribofuranosyl-based linkages.

One embodiment of the invention is a method of treating an inflammatory disease or condition by administering SSOs to a patient or a live subject. The SSOs that are administered alter the splicing of a pre-mRNA to produce a splice variant that encodes a stable, secreted, ligand-binding form of a receptor of the TNFR superfamily, thereby decreasing the activity of the ligand for that receptor. In another embodiment, the invention is a method of producing a stable, secreted, ligand-binding form of a receptor of the TNFR superfamily in a cell by administering SSOs to the cell.

The foregoing and other objects and aspects of the present invention are discussed in detail in the drawings herein and the specification set forth below.

### BRIEF DESCRIPTION OF THE DRAWINGS

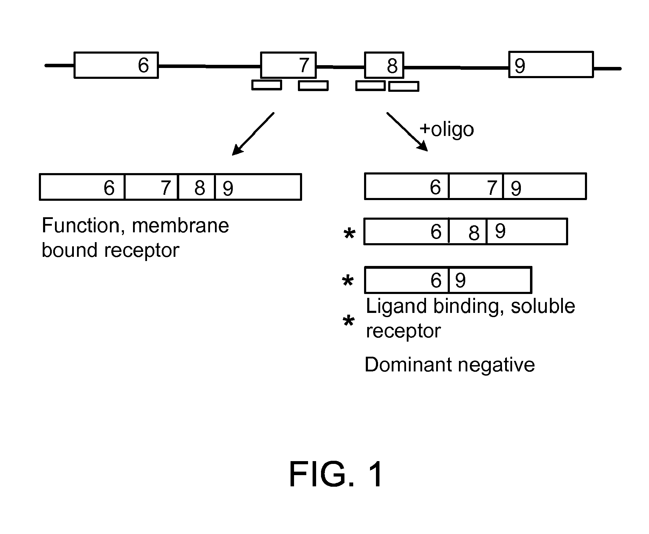

FIG. 1 depicts the structure of a portion of the tumor necrosis factor receptor pre-mRNA and spliced products for TNFR1 and TNFR2. These transcripts normally contain exon 7 and exon 8, which code for the transmembrane domain of the receptors. SSOs (bars) directed towards either or both of these exons elicit alternative splicing events, resulting in transcripts that lack the full transmembrane domain.

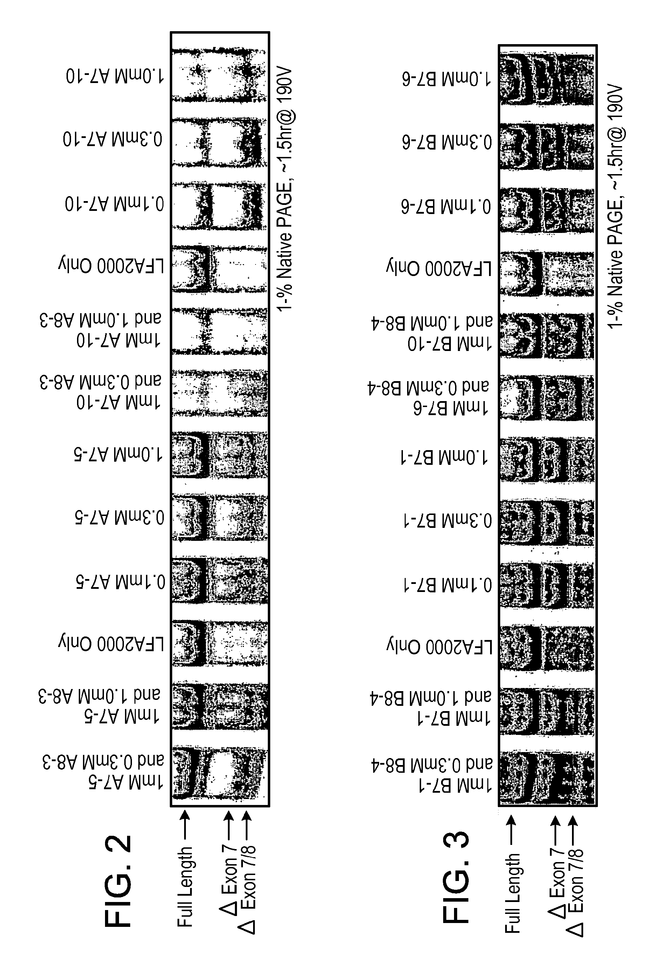

FIG. 2 shows the splicing products of SSOs for murine TNFR1 in cell culture. NIH-3T3 cells were mock transfected [Lipofectamine^{®} 2000 (LFA2000 Only)] or transfected with the indicated concentration of either an exon 7 skipping TNFR1 SSO, A7-5 or A7-10, alone or a combination of exon 7 skipping SSO and an exon 8 skipping SSO, A8-3. Total RNA was isolated and RT-PCR performed 24 hours later. The PCR primers were used to amplify from Exon 5 to Exon 9, so that "Full Length" TNFR1 is represented by a 475 bp band. Transcripts lacking exon 7 (Δ Exon 7) and lacking both exon 7 and exon 8 (Δ Exon 7/8) are represented by 361 bp and 332 bp bands, respectively.

FIG. 3 shows the splicing products of SSOs for murine TNFR2 in cell culture. NIH-3T3 cells were mock transfected (LFA2000 Only) or transfected with the indicated concentration of either an exon 7 skipping TNFR2 SSO, B7-6 or B7-1, alone or a combination of exon 7 skipping oligonucleotide and an exon 8 skipping oligonucleotide, B8-4. Total RNA was isolated and RT-PCR performed 24 hours later. The PCR primers were used to amplify from Exon 5 to Exon 9, so that "Full Length" TNFR2 is represented by a 486 bp band. Transcripts lacking exon 7 (Δ Exon 7) and lacking both exon 7 and exon 8 (Δ Exon 7/8) are represented by 408 bp and 373 bp bands, respectively.

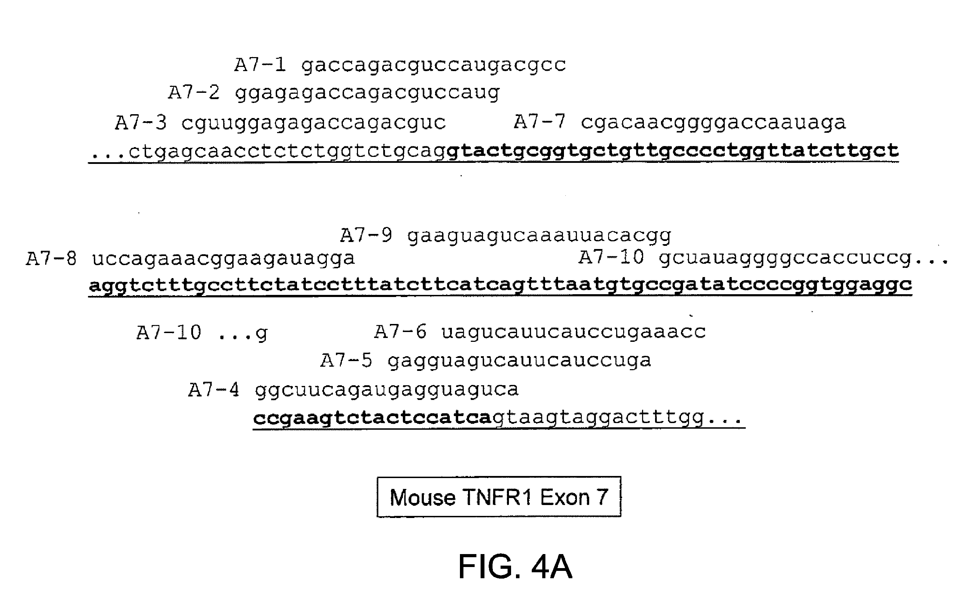

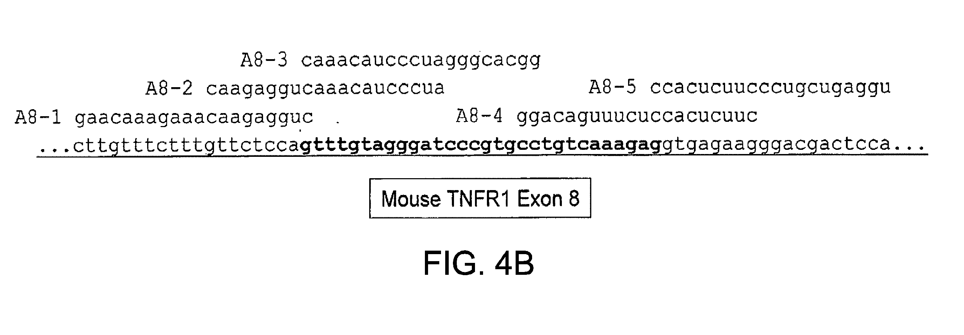

FIGs. 4A and 4B present the sequences of exons 7 (4A) and 8 (4B) of murine TNFR1 and of the flanking introns. Also shown are the sequences of 2'O-Me-oligoribonucleoside-phosphorothioate SSOs that were assayed for splice switching activity.

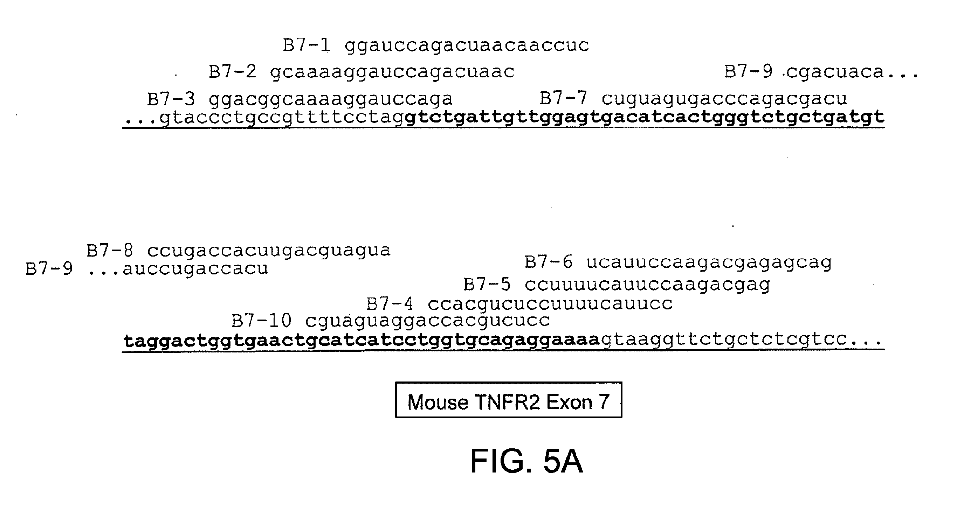

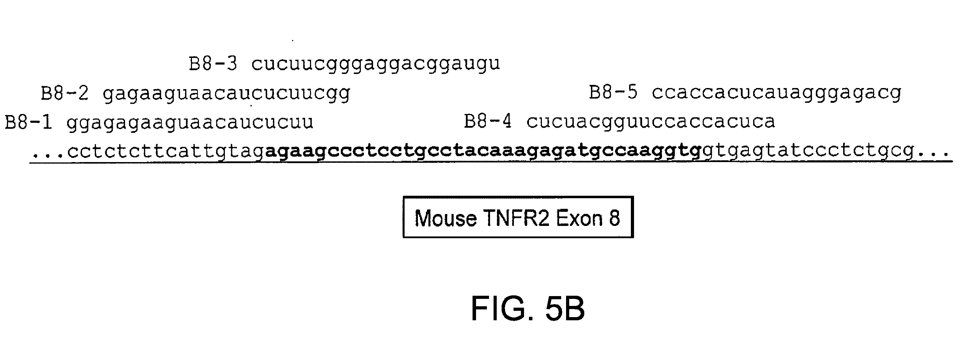

FIGs. 5A and 5B present the sequences of exons 7 (5A) and 8 (5B) of murine TNFR2 and of the flanking introns. Also shown are the sequences of 2'O-Me-oligoribonucleoside-phosphorothioate SSOs that were assayed for splice switching activity.

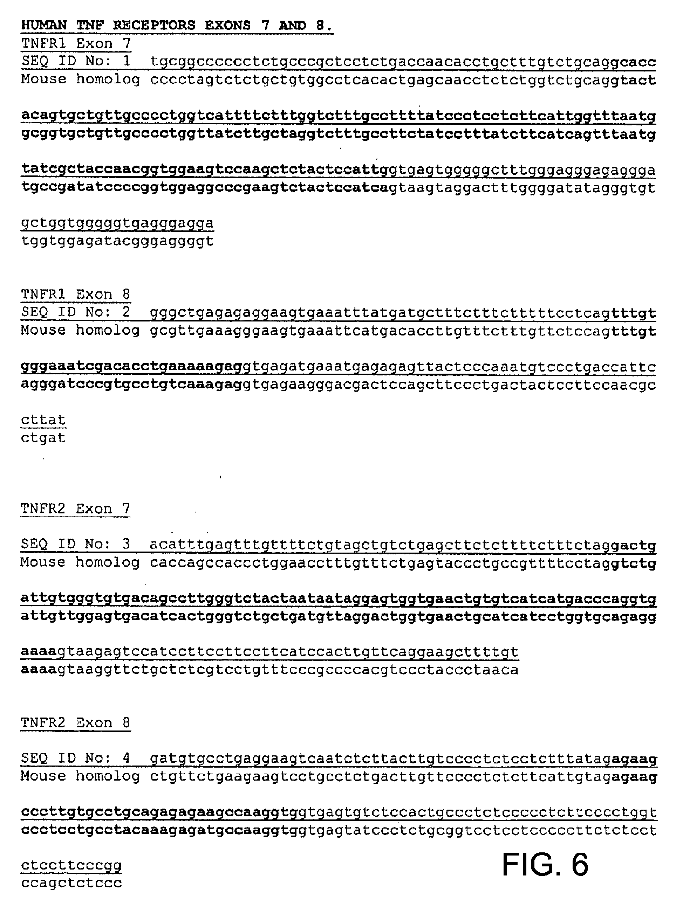

FIG. 6 provides an alignment of the human and murine TNF receptor genes in the regions that encode the transmembrane exons. The murine sequences, SEQ ID Nos: 107, 108, 109, and 110, are homologous to the human sequences, SEQ ID Nos: 1, 2, 3, and 4, respectively.

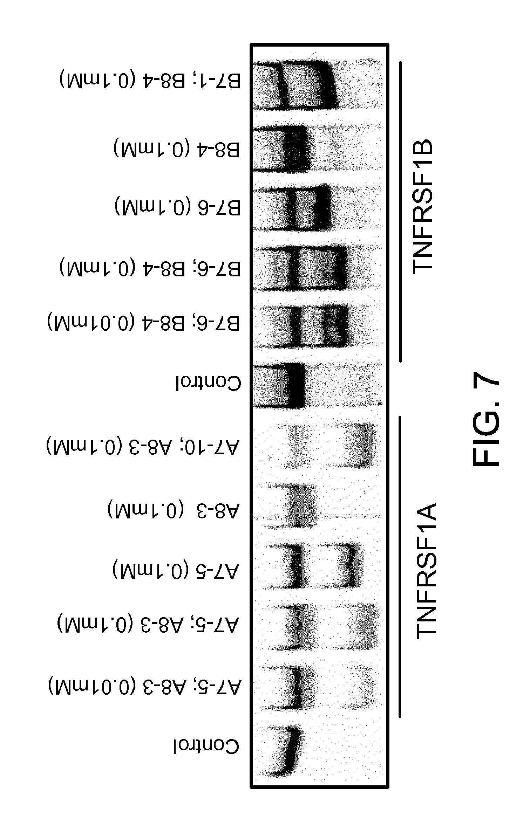

FIG. 7 shows the splicing products of SSOs for primary mouse hepatocyte cultures, in assays conducted as described in Figures 2 and 3.

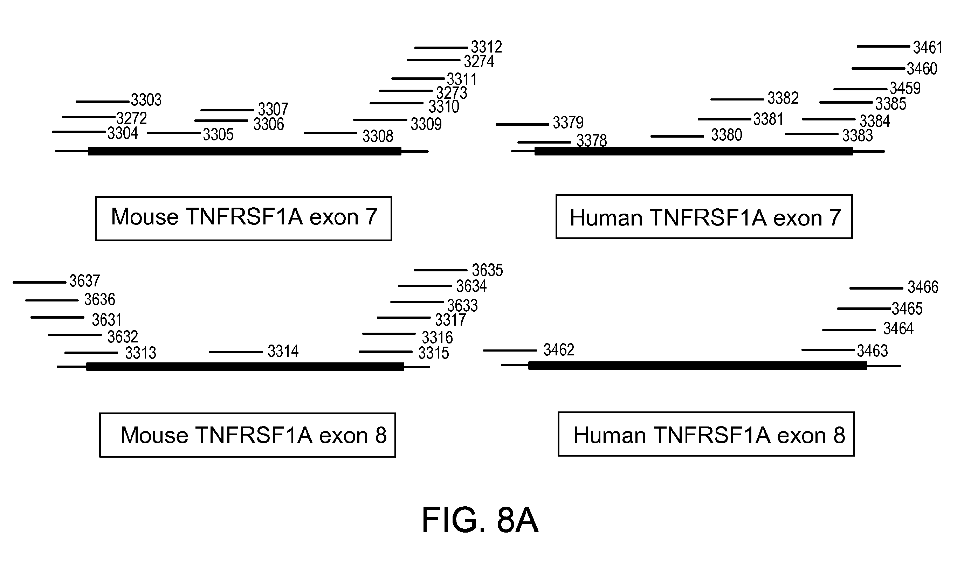

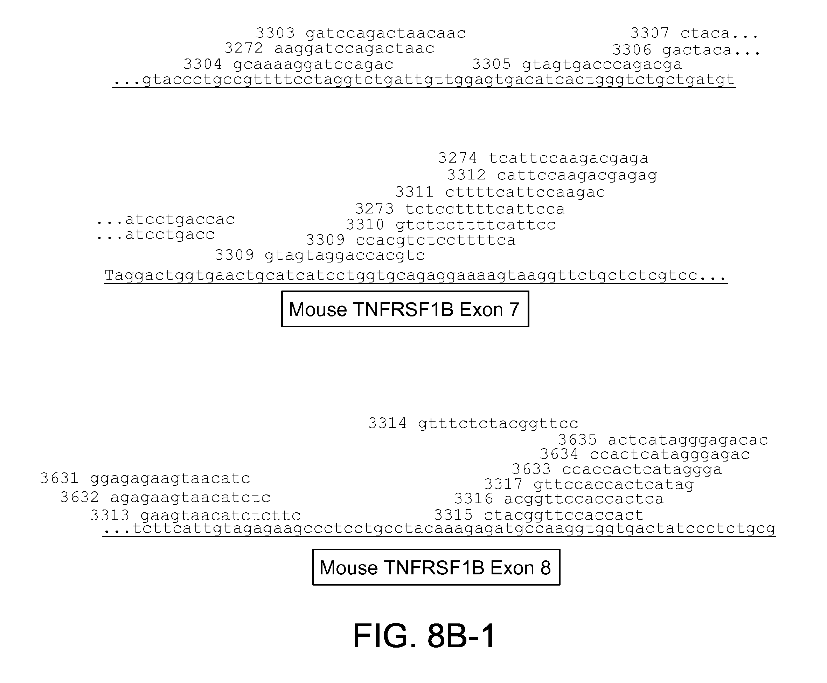

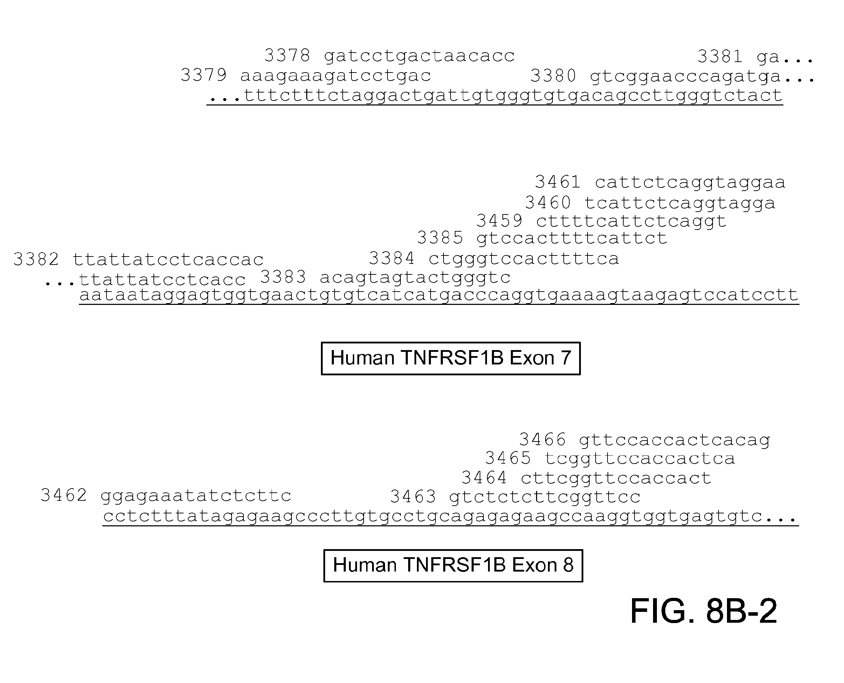

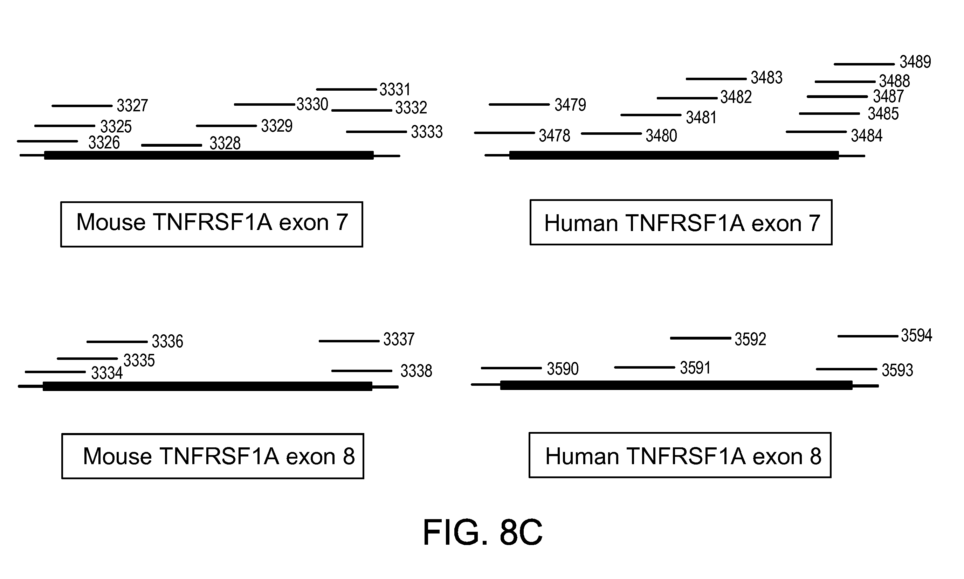

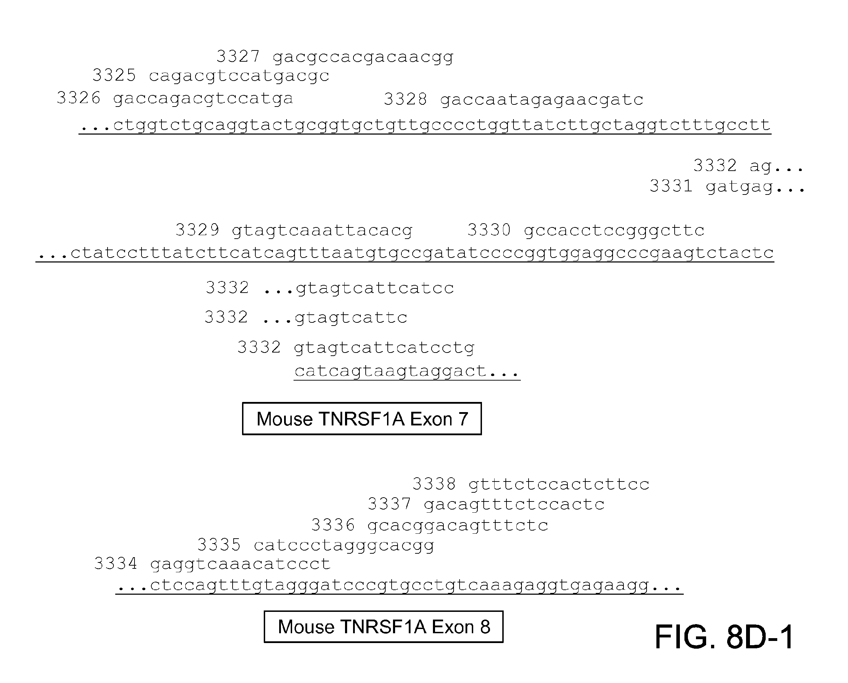

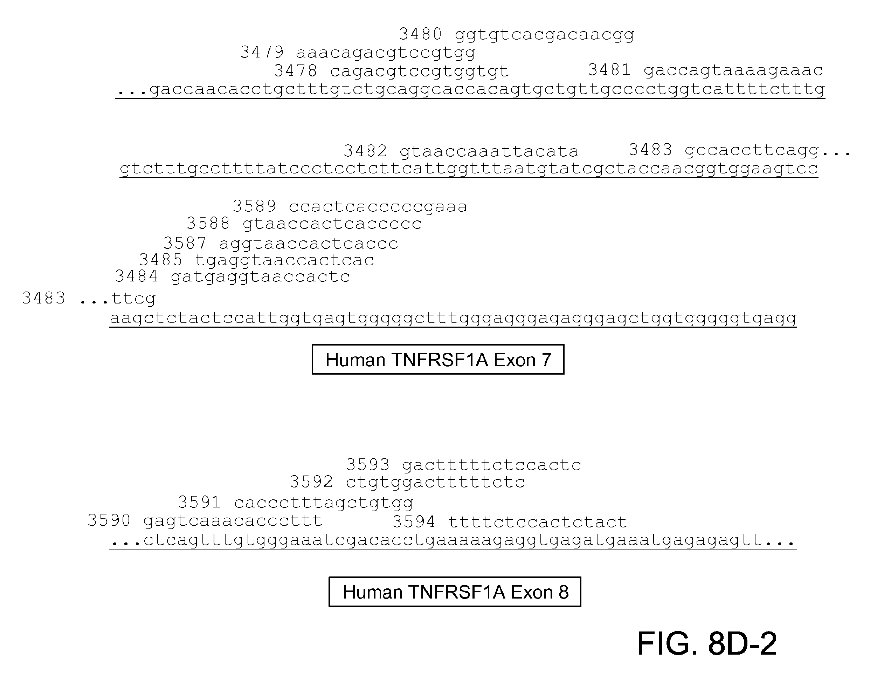

FIGs. 8A-8D provide mouse and human TNFR2 (TNFRSF1B) (8A and 8B) and TNFR1 (TNFRSF1A) (8C and 8D) LNA SSO sequences from Tables 2 and 3. Figures 8A and 8C schematically illustrate the position of each SSO relative to the targeted exon. Figures 8B and 8D show the pre-mRNA sequence (5' to 3') and the SSOs (3' to 5') hybridized to it.

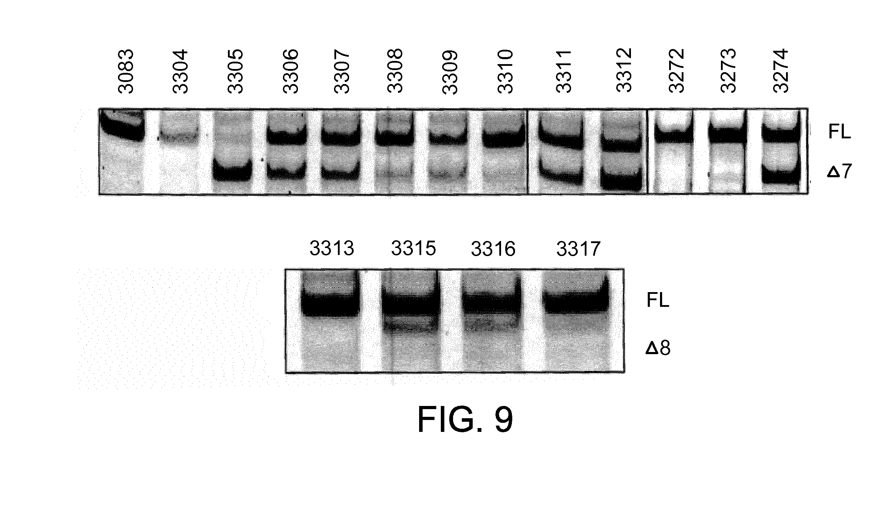

FIG. 9 shows the splicing products for L929 murine cells treated with LNA SSOs. Cells were transfected with the indicated LNA SSO at a final concentration of 50 nM. After 24 hours, the cells were lysed and analyzed for splice switching by RT-PCR. Top panel, SSOs targeted to exon 7; bottom panel, SSOs targted to exon 8. FL, full length TNFR2 amplicon; Δ7, Δ8, Δ7/8, amplicons of the respective TNFR2 splice variants.

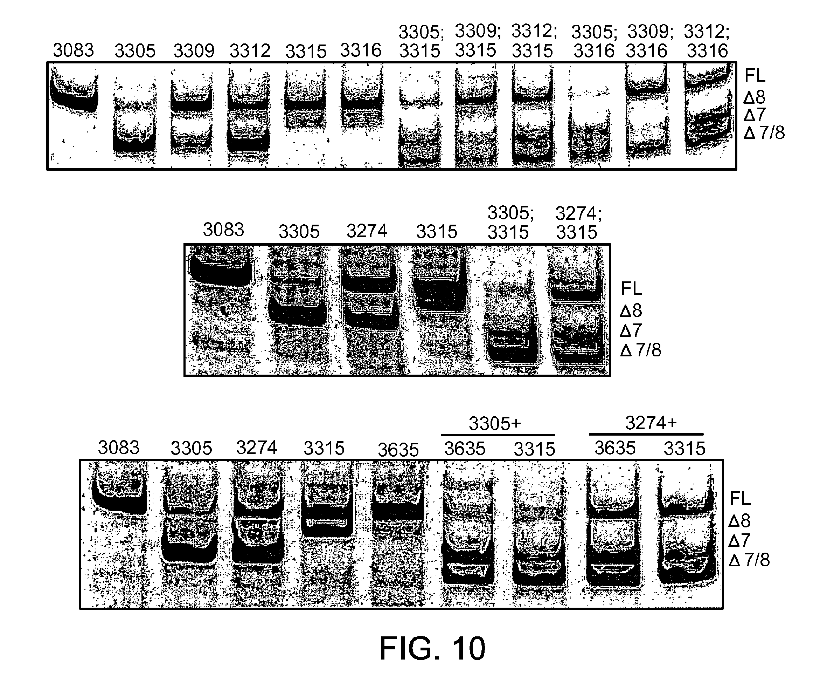

FIG. 10 shows the splicing products for L929 murine cells using LNA SSO combinations targeted to TNFR2. L929 cells were treated with the indicated single or multiple LNA SSOs at 50 nM each and analyzed 24 hours later as described in Figure 9.

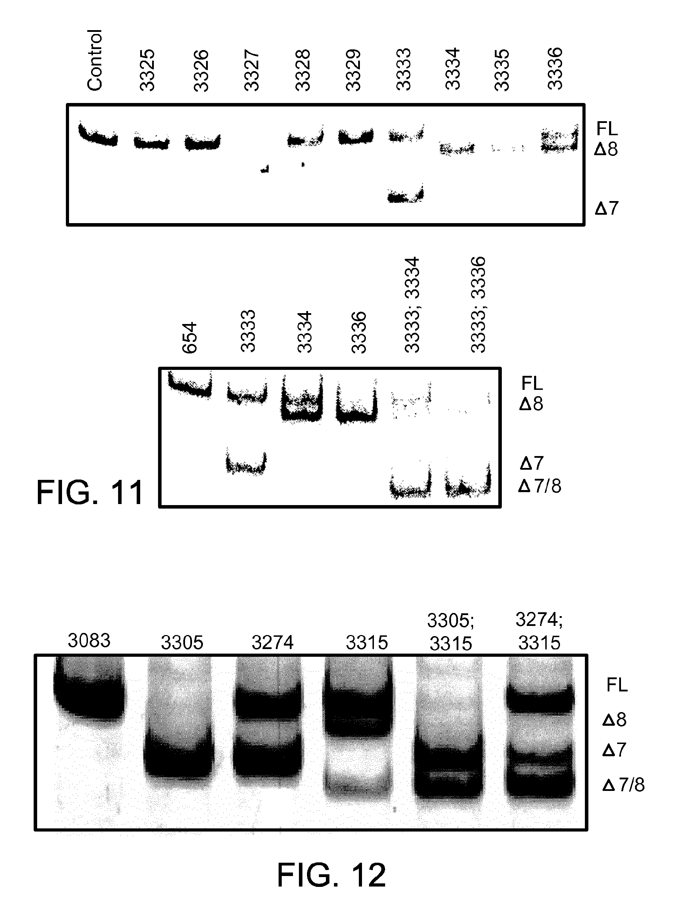

FIG. 11 the splicing products for L929 murine cells using LNA SSO combinations targeted to TNFR1. L929 cells were treated with the indicated single or multiple LNA SSOs at 50 nM each and analyzed 24 hours later as described in Figure 9.

FIG. 12 shows the splicing products for primary mouse hepatocytes treated with LNA SSOs. Primary mouse hepatocytes were transfected with 33 nM each final concentration of the indicated single or multiple LNA SSOs and analyzed as described in Figure 9.

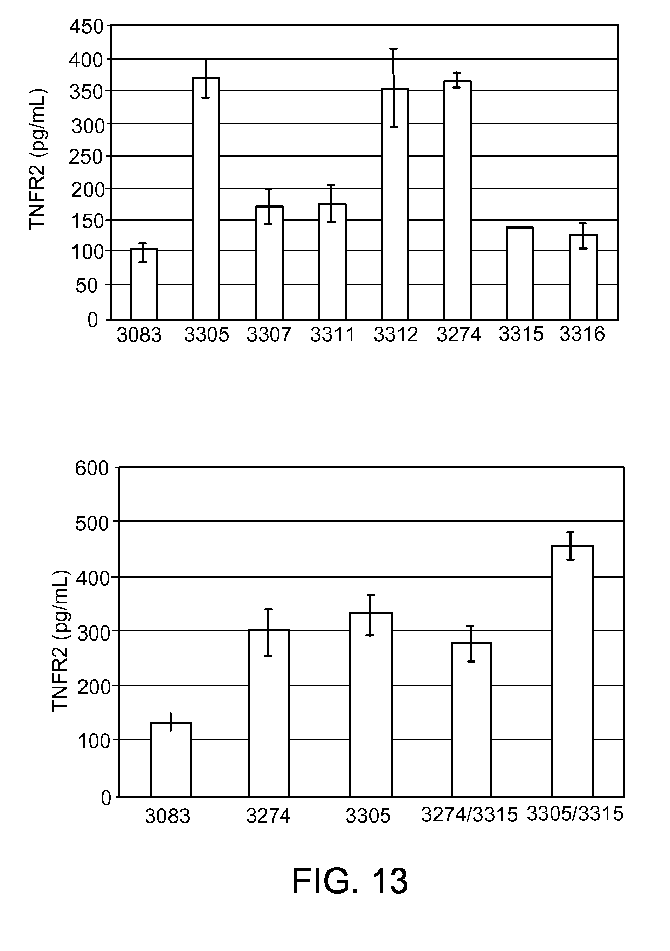

FIG. 13 graphically illustrates detection of secreted TNFR2 splice variants from L929 cells (left) and primary mouse hepatocytes (right). Cells were transfected with the indicated LNA SSOs. After 72 hours, the extracellular media was removed and analyzed by enzyme linked immunosorbant assay (ELISA) using antibodies from the Quantikine® Mouse sTNF RII ELISA kit from R&D Systems (Minneapolis, MN). The data are expressed as pg soluble TNFR2 per mL.

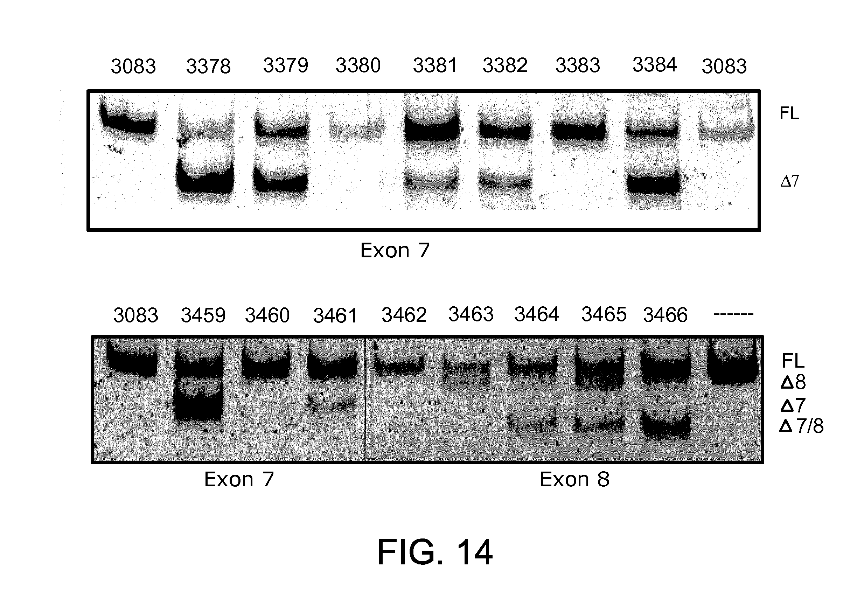

FIG. 14 shows the splicing products for primary human hepatocytes treated with LNA SSOs targeted to TNFR2. Primary human hepatocytes were transfected with the indicated LNA SSO and analyzed for splice switching by RT-PCR after 24 hours as described in Figure 9. The PCR primers were used to amplify from Exon 5 to Exon 9, so that "Full Length"(FL) TNFR2 is represented by a 463 bp band. Transcripts lacking exon 7 (Δ Exon 7), lacking exon 8 (Δ Exon 8), and lacking both exon 7 and exon 8 (Δ exon 7/8) are represented by 385 bp, 428 bp, and 350 bp bands, respectively.

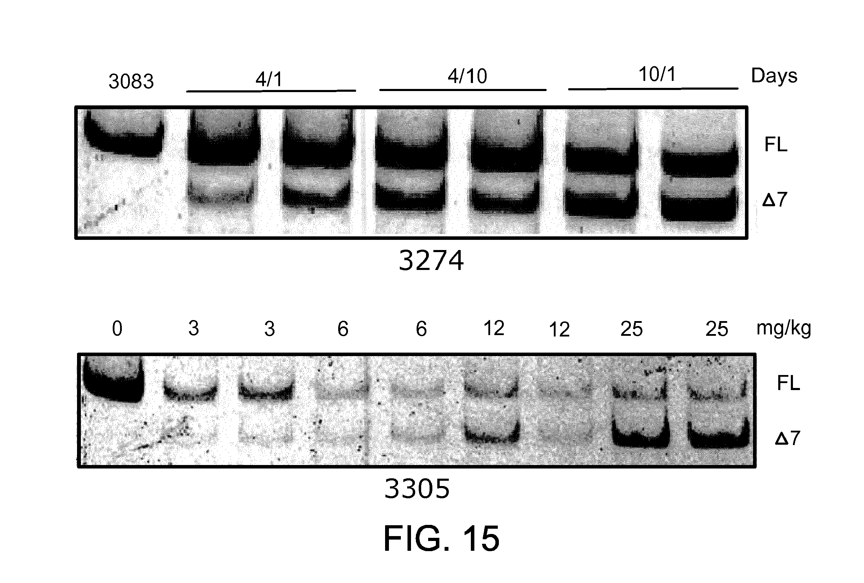

FIG. 15 shows the splicing products for intraperitoneal (i.p.) injection of LNA 3274 (top) and 3305 (bottom) in mice. LNA 3274 was injected i.p. at 25mg/kg/day for either 4 days (4/1 and 4/10) or 10 days (10/1). Mice were sacrificed either 1 day (4/1 and 10/1) or 10 (4/10) days after the last injection and total RNA from liver was analyzed for splice switching of TNFR2 by RT-PCR. LNA 3305 was injected at the indicated dose per day for 4 days. Mice were sacrificed the next day and the livers analyzed as with 3274 treated animals.

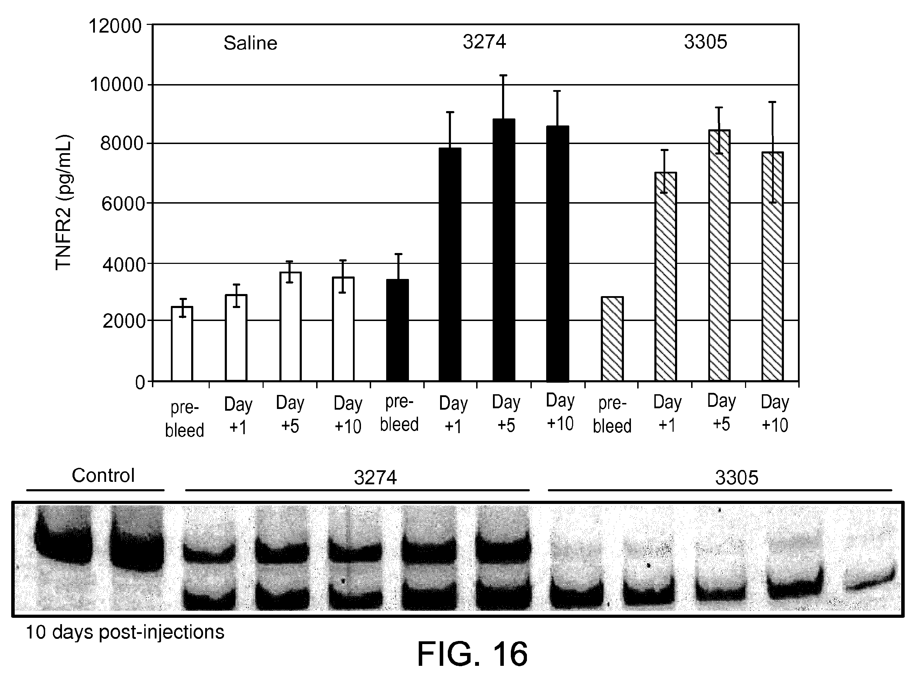

FIG. 16 (top panel) graphically illustrates the amount of soluble TNFR2 in mouse serum 10 days after SSO treatment. Mice were injected i.p. with the indicated SSO or saline (n=5 per group) at 25 mg/kg/day for 10 days. Serum collected 4 days before injections began and the indicated number of days after the last injection. Sera was analyzed by ELISA as described in Figure 13. At day 10, mice were sacrificed and livers were analyzed for TNFR2 splice switching by RT-PCR (bottom panel) as described in Figure 9.

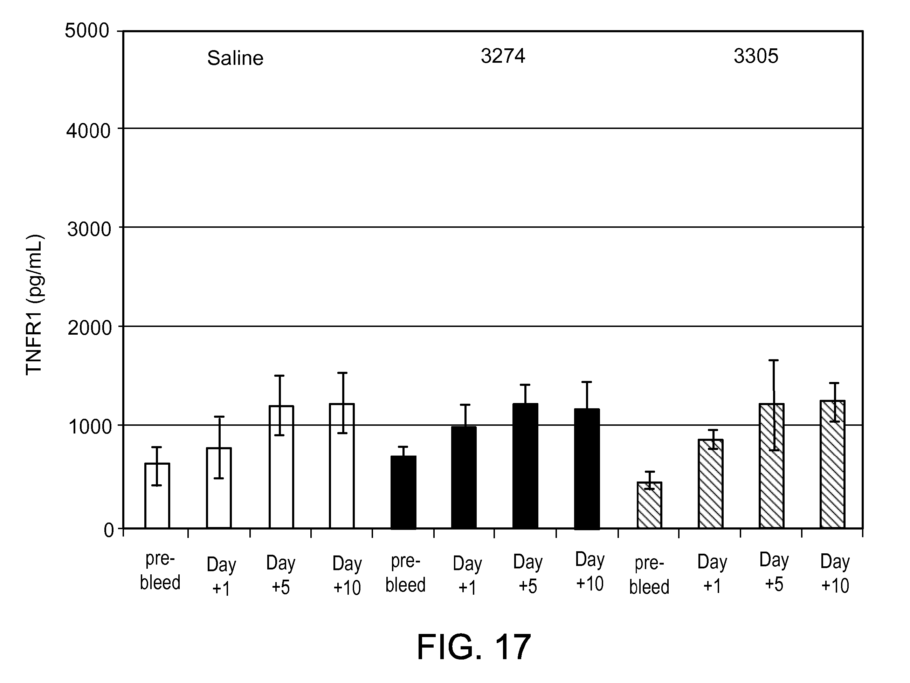

FIG. 17 graphically illustrates the amount of soluble TNFR1 in the serum after TNFR2 SSO treatment. Mouse serum from Figure 16 was analyzed for soluble TNFR1 by ELISA using antibodies from the Quantikine® Mouse sTNF RI ELISA kit from R&D Systems (Minneapolis, MN).

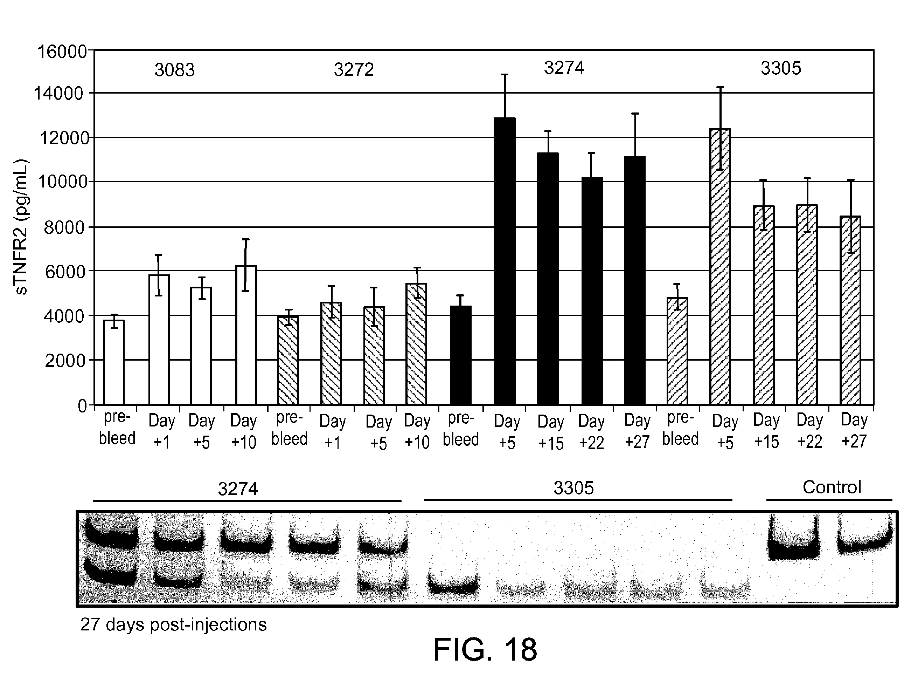

FIG. 18 (top panel) graphically illustrates the amount of soluble TNFR2 in mouse serum 27 days after SSO treatment. Mice were treated as in Figure 16, except that serum samples were collected until day 27 after the last injection. LNA 3083 and 3272 are control SSOs with no TNFR2 splice switching ability. At day 27, mice were sacrificed and livers were analyzed for TNFR2 splice switching by RT-PCR (bottom panel) as described in Figure 9.

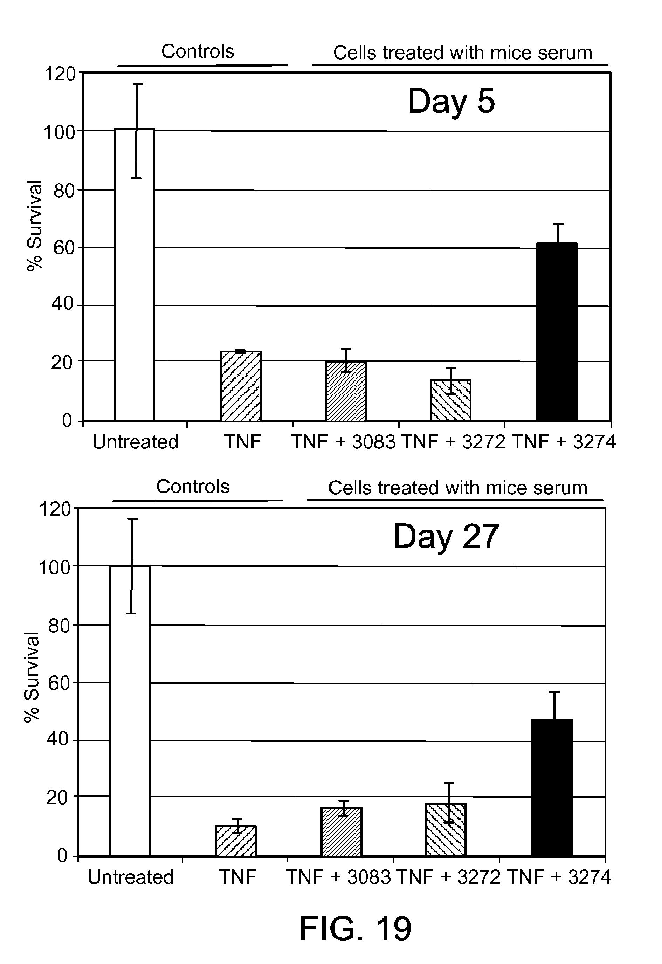

FIG. 19 graphically depicts the anti-TNF-α activity in serum from LNA oligonucleotide-treated mice. L929 cells were treated with either 0.1 ng/mL TNF-α (TNF), or TNF-α plus 10% serum from mice treated with the indicated oligonucleotide (see also Figure 18). Cell viability was measured 24 hours later and normalized to untreated cells (Untreated).

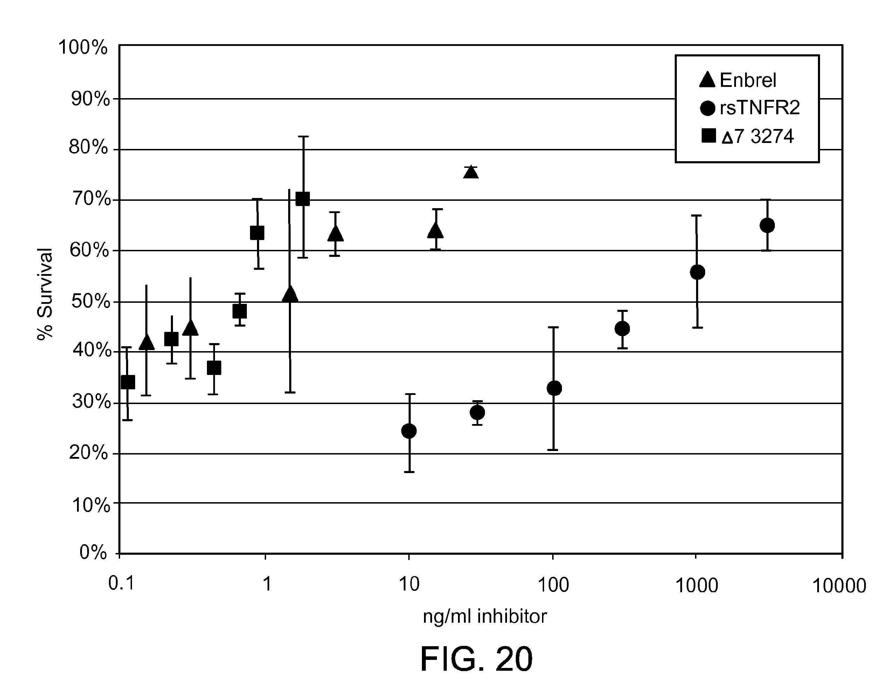

FIG. 20 graphically compares the anti-TNF-α activity of serum from LNA oligonucleotide-treated mice to recombinant soluble TNFR2 (rsTNFR2) and to that of Enbrel® using the cell survival assay described in Figure 19.

### DETAILED DESCRIPTION OF THE INVENTION

As used herein, the terms "tumor necrosis factor receptor superfamily" or "TNFR superfamily" or "TNFRSF" refer to a group of type I transmembrane proteins, with a carboxy-terminal intracellular domain and an amino-terminal extracellular domain characterized by a common cysteine rich domain (CRD). The TNFR superfamily consists of receptors, mediate cellular signaling as a consequence of binding to one or more ligands in the TNF superfamily. The TNFR superfamily can be divided into two subgroups: receptors containing the intracellular death domain (DD) and those lacking it. The DD is an 80 amino acid motif that is responsible for the induction of apoptosis following receptor activation. Members of the TNFR superfamily include, but are not limited to, TNFR1 (TNFRSF1A), TNFR2 (TNFRSF1B), RANK (TNFRSF11A), CD40 (TNFRSF5), CD30 (TNFRSF8), and LT-βR (TNFRSF3).

As used herein, the terms "tumor necrosis factor superfamily" or "TNF superfamily" refer to the group of ligands that bind to one or more receptors in the TNFR superfamily. The binding of a TNF family ligand to its corresponding receptor or receptors mediate cellular signaling. Members of the TNF superfamily include, but are not limited to, TNF-α, RANKL, CD40L, LT-α, or LT-β.

As used herein, the term "an inflammatory disease or condition" refers to a disease, disorder, or other medical condition that at least in part results from or is aggravated by the binding of a ligand from the TNF superfamily to its corresponding receptor or receptors. Such diseases or conditions include, but are not limited to, those associated with increased levels of the TNF superfamily ligand, increased levels of TNFR superfamily receptor levels, or increased sensitization of the corresponding signaling pathway. Examples of inflammatory diseases or conditions include, but are not limited to, rheumatoid arthritis, juvenile rheumatoid arthritis, psoriasis, psoriatic arthritis, ankylosing spondylitis, inflammatory bowel disease (including Crohn's disease or ulcerative colitis), hepatitis, sepsis, alcoholic liver disease, and non-alcoholic steatosis.

As used herein, the term "hepatitis" refers to a gastroenterological disease, condition, or disorder that is characterized, at least in part, by inflammation of the liver. Examples of hepatitis include, but are not limited to, hepatitis associated with hepatitis A virus, hepatitis B virus, hepatitis C virus, or liver inflammation associated with ischemia/reperfusion.

As used herein, the terms "membrane bound form" or "integral membrane form" refer to proteins having amino acid sequences that span a cell membrane, with amino acid sequences on each side of the membrane.

As used herein, the term "stable, secreted, ligand-binding form" or as it is sometimes known "stable, soluble, ligand-binding form." (where the terms "secreted" and "soluble" are synonymous and interchangeable herein) refer to proteins that are related to the native membrane bound form receptors, in such a way that they are secreted and stable and still capable of binding to the corresponding ligand. It should be noted that these forms are not defined by whether or not such secreted forms are physiological, only that the products of such splice variants would be secreted, stable, and still capable of ligand-binding when produced.

The term "secreted" means that the form is soluble, i.e., that it is no longer bound to the cell membrane. In this context, a form will be soluble if using conventional assays known to one of skill in the art most of this form can be detected in fractions that are not associated with the membrane, e.g., in cellular supernatants or serum.

The term "stable" means that the secreted form is detectable using conventional assays by one of skill in the art. For example, western blots, ELISA assays can be used to detect the form from harvested cells, cellular supernatants, or serum from patients.

The term "ligand-binding" means that the form retains at least some significant level, although not necessarily all, of the specific ligand-binding activity of the corresponding integral membrane form.

As used herein, the term "to reduce the activity of a ligand" refers to any action that leads to a decrease in transmission of an intracellular signal resulting from the ligand binding to or interaction with the receptor. For example, activity can be reduced by binding of the ligand to a soluble form of its receptor or by decreasing the quantity of the membrane form of its receptor available to bind the ligand.

As used herein, the term "altering the splicing of a pre-mRNA" refers to altering the splicing of a cellular pre-mRNA target resulting in an altered ratio of splice products. Such an alteration of splicing can be detected by a variety of techniques well known to one of skill in the art. For example, RT-PCR on total cellular RNA can be used to detect the ratio of splice products in the presence and the absence of an SSO.

As used herein, the term "complementary" is used to indicate a sufficient degree of complementarity or precise pairing such that stable and specific binding occurs between an SSO and a DNA or RNA containing the target sequence. It is understood in the art that the sequence of an SSO need not be 100% complementary to that of its target. There is a sufficient degree of complementarity when, under conditions which permit splicing, binding to the target will occur and non-specific binding will be avoided.

The present invention employs splice switching oligonucleotides or splice switching oligomers (SSOs) to control the alternative splicing of receptors from the TNFR superfamily so that the amount of a soluble, stable, secreted, ligand-binding form is increased and the amount of the integral membrane form is decreased. The methods and compositions of the present invention can be used in the treatment of diseases associated with excessive TNF superfamily activity.

Accordingly one embodiment of the invention is a method of treating an inflammatory disease or condition by administering SSOs to a patient. The SSOs that are administered after the splicing of a pre-mRNA to produce a splice variant that encodes a stable, secreted, ligand-binding form of a receptor of the TNFR superfamily, thereby decreasing the activity of the ligand for that receptor. In another embodiment, the invention is a method of producing a stable, secreted, ligand-binding form of a receptor of the TNFR superfamily in a cell by administering SSOs to the cell.

The following aspects of the present invention discussed below apply to the foregoing embodiments.

The length of the SSO is similar to an antisense oligonucleotide (ASON), typically between about 10 and 24 nucleotides. The invention can be practiced with SSOs of several chemistries that hybridize to RNA, but that do not activate the destruction of the RNA by RNase H, as do conventional antisense 2'-deoxy oligonucleotides. The invention can be practiced using 2'O modified nucleic acid oligomers, such as 2'O-methyl or 2'O-methyloxyethyl phosphorothioate. The nucleobases do not need to be linked to sugars; so-called peptide nucleic acid oligomers or morpholine-based oligomers can be used. A comparison of these different linking chemistries is found in Sazani, P. et al., 2001, Nucleic Acids Res. 29:3695. The term splice-switching oligonucleotide is intended to cover the above forms. Those skilled in the art will appreciate the relationship between antisense oligonucleotide gapmers and SSOs. Gapmers are ASON that contain an RNase H activating region (typically a 2'-deoxyribonucleoside phosphorothioate) which is flanked by non-activating nuclease resistant oligomers. In general, any chemistry suitable for the flanking sequences in a gapmer ASON can be used in an SSO.

The SSOs of this invention may be made through the well-known technique of solid phase synthesis. Any other means for such synthesis known in the art may additionally or alternatively be used. It is well known to use similar techniques to prepare oligonucleotides such as the phosphorothioates and alkylated derivatives.

A particularly preferred chemistry is provided by locked nucleic acids (LNA) (Koshkin, A.A., et al., 1998, Tetrahedron 54:3607; Obika, S., et al., 1998, Tetrahedron Lett. 39:5401). LNA are conventional phosphodiester-linked ribonucleotides, except the ribofuranosyl moiety is made bicyclic by a bridge between the 2'O and the 4'C. This bridge constrains the conformation of ribofuranosyl ring into the conformation, the 3'*-endo* conformation, which is adopted when a oligonucleotide hybridizes to a complementary RNA. Recent advances in the synthesis of LNA are described in WO 03/095467. The bridge is most typically a methylene or an ethylene. The synthesis of 2'O,4'C-ethylene-bridged nucleic acids (ENA), as well as other LNA, is described in Morita, et al., 2003, Bioorg. & Med. Chem. 11:2211. However, alternative chemistries can be used and the 2'O may be replaced by a 2'N. LNA and conventional nucleotides can be mixed to form a chimeric SSO. For example, chimeric SSO of alternating LNA and 2'deoxynucleotides or alternating LNA and 2'O-Me or 2'O-MOE can be employed. An alternative to any of these chemistries, not merely the 2'-deoxynucleotides, is a phosphorothioatediester linkage replacing phosphodiester. For *in vivo* use, phosphorothioate linkages are preferred.

When LNA nucleotides are employed in an SSO it is preferred that non-LNA nucleotides also be present. LNA nucleotides have such high affinities of hybridization that there can be significant non-specific binding, which may reduce the effective concentration of the free-SSO. When LNA nucleotides are used they may be alternated conveniently with 2'-deoxynucleotides. The pattern of alternation is not critical. Alternating nucleotides, alternating dinucleotides or mixed patterns, e.g., LDLDLD or LLDLLD or LDDLDD can be used. When 2'-deoxynucleotides or 2'-deoxynucleoside phosphorothioates are mixed with LNA nucleotides it is important to avoid RNase H activation. It is expected that between about one third and two thirds of the LNA nucleotides of an SSO will be suitable. For example if the SSO is a 12-mer, then at least four LNA nucleotides and four conventional nucleotides will be present.

The bases of the SSO may be the conventional cytosine, guanine, adenine and uracil or thymidine. Alternatively modified bases can also be used. Of particular interest are modified bases that increase binding affinity. One non-limiting example of preferred modified bases are the so-called G-clamp or 9-(aminoethoxy)phenoxazine nucleotides, cytosine analogs that form 4 hydrogen bonds with guanosine. (Flanagan, W.M., et al., 1999, Proc. Natl. Acad. Sci. 96:3513; Holmes, S.C., 2003, Nucleic Acids Res. 31:2759).

Numerous alternative chemistries which do not activate RNase H are available. For example, suitable SSOs may be oligonucleotides wherein at least one, or all, of the internucleotide bridging phosphate residues are modified phosphates, such as methyl phosphonates, methyl phosphonothioates, phosphoromorpholidates, phosphoropiperazidates, and phosphoroamidates. For example, every other one of the internucleotide bridging phosphate residues may be modified as described. In another non-limiting example, such SSO are oligonucleotides wherein at least one, or all, of the nucleotides contain a 2' loweralkyl moiety (e.g., C1-C4, linear or branched, saturated or unsaturated alkyl, such as methyl, ethyl, ethenyl, propyl, 1-propenyl, 2-propenyl, and isopropyl). For example, every other one of the nucleotides may be modified as described. [See references in U.S. Pat. 5,976,879 col. 4].

The length of the SSO (i.e. the number of monomers in the oligomer) will be from about 10 to about 30 bases in length. In one embodiment, 20 bases of 2'*O*-Me-ribonucleosides phosphorothioates are effective. Those skilled in the art appreciate that when affinity-increasing chemical modifications are used, the SSO can be shorter and still retain specificity. Those skilled in the art will further appreciate that an upper limit on the size of the SSO is imposed by the need to maintain specific recognition of the target sequence, and to avoid secondary-structure forming self hybridization of the SSO and by the limitations of gaining cell entry. These limitations imply that an SSO of increasing length (above and beyond a certain length which will depend on the affinity of the SSO) will be more frequently found to be less specific, inactive or poorly active.

SSOs of the invention include, but are not limited to, modifications of the SSO involving chemically linking to the SSO one or more moieties or conjugates which enhance the activity, cellular distribution or cellular uptake of the SSO. Such moieties include, but are not limited to, lipid moieties such as a cholesterol moiety, cholic acid, a thioether, e.g. hexyl-S-tritylthiol, a thiocholesterol, an aliphatic chain, e.g., dodecandiol or undecyl residues, a phospholipids, e.g., di-hexadecyl-rac-glycerol or triethylammonium 1,2-di-O-hexadecyl-racglycero-3-H-phosphonate, a polyamine or a polyethylene glycol chain, an adamantane acetic acid, a palmityl moiety, an octadecylamine or hexylamino-carbonyl-oxycholesterol moiety.

It is not necessary for all positions in a given SSO to be uniformly modified, and in fact more than one of the aforementioned modifications may be incorporated in a single compound or even at a single nucleoside within an SSO.

The SSOs may be admixed, encapsulated, conjugated, or otherwise associated with other molecules, molecule structures, or mixtures of compounds, as for example liposomes, receptor targeted molecules, oral, rectal, topical or other formulation, for assisting in uptake, distribution, and/or absorption.

Those skilled in the art appreciate that cellular differentiation includes, but is not limited to, differentiation of the spliceosome. Accordingly, the activity of any particular SSO of the invention can depend upon the cell type into which they are introduced. For example, SSOs which are effective in cell type may be ineffective in another cell type.

The methods, oligonucleotides, and formulations of the present invention are also useful as *in vitro* or *in vivo* tools to examine splicing in human or animal genes. Such methods can be carried out by the procedures described herein, or modifications thereof which will be apparent to skilled persons.

The invention can be used to treat any condition in which the medical practitioner intends to limit the effect of a TNF superfamily ligand or the signalling pathway activated by such ligand. In particular, the invention can be used to treat an inflammatory disease. In one embodiment, the condition is an inflammatory systemic disease, e.g., rheumatoid arthritis or psoriatic arthritis. In another embodiment, the disease is an inflammatory liver disease. Examples of inflammatory liver diseases include, but are not limited to, hepatitis associated with the hepatitis A, B, or C viruses, alcoholic liver disease, and non-alcoholic steatosis. In yet another embodiment, the inflammatory disease is a skin condition such as psoriasis.

The uses of the present invention include, but are not limited to, treatment of diseases for which known TNF antagonists have been shown useful. Three specific TNF antagonists are currently FDA-approved. The drugs are etanercept (Enbrel®), infliximab (Remicade®) and adalimumab (Humira®). One or more of these drugs is approved for the treatment of rheumatoid arthritis, juvenile rheumatoid arthritis, psoriasis, psoriatic arthritis, ankylosing spondylitis, and inflammatory bowel disease (Crohn's disease or ulcerative colitis).

In a preferred embodiment, the receptor is either the TNFR1 or TNFR2 receptors. In other embodiments, the receptor is a member of the TNFR superfamily that is sufficiently homologous to TNFR1 and TNFR2, e.g., TNFRSF3, TNFRSF5, or TNFRSF11A, so that deletion of either or both exons homologous to exons 7 and 8 results in a secreted form. Those skilled in the art appreciate that the operability of the invention is not determined by whether or not such secreted forms are physiological, only that the products of such splice variants are secreted, stable, and capable of ligand-binding.

The administration of the SSO to subjects can be accomplished using procedures developed for ASON. ASON have been successfully administered to experimental animals and human subjects by intravenous administration in saline in doses as high as 6 mg/kg three times a week (Yacysyhn, B.R., et al., 2002, Gut 51:30 (anti-ICAM-1 ASON for treatment of Crohn's disease); Stevenson, J., et al., 1999, J. Clinical Oncology 17:2227 (anti-RAF-1 ASON targeted to PBMC)). The pharmacokinetics of 2'O-MOE phosphorothioate ASON, directed towards TNF-α has been reported (Geary, R.S., et al., 2003, Drug Metabolism and Disposition 31:1419). The systemic efficacy of mixed LNA/DNA molecules has also been reported (Fluiter, K., et al., 2003, Nucleic Acids Res. 31:953).

The systemic activity of SSO in a mouse model system was investigated using 2'O-MOE phosphorothioates and PNA chemistries. Significant activity was observed in all tissues investigated except brain, stomach and dermis (Sazani, P., et al., 2002, Nature Biotechnology 20, 1228).

In general any method of administration that is useful in conventional antisense treatments can be used to administer the SSO of the invention. For testing of the SSO in cultured cells, any of the techniques that have been developed to test ASON or SSO may be used.

Formulations of the present invention comprise SSOs in a physiologically or pharmaceutically acceptable carrier, such as an aqueous carrier. Thus formulations for use in the present invention include, but are not limited to, those suitable for parenteral administration including intraperitoneal, intravenous, intraarterial, subcutaneous, or intramuscular injection or infusion., as well as those suitable topical (including ophthalmic and to mucous membranes including vaginal delivery), oral, rectal or pulmonary (including inhalation or insufflation of powders or aerosols, including by nebulizer, intratracheal, intranasal delivery) administration. The formulations may conveniently be presented in unit dosage form and may be prepared by any of the methods well known in the art. The most suitable route of administration in any given case may depend upon the subject, the nature and severity of the condition being treated, and the particular active compound which is being used.

Pharmaceutical compositions of the present invention include, but are not limited to, the physiologically and pharmaceutically acceptable salts thereof: i.e, salts that retain the desired biological activity of the parent compound and do not impart undesired toxicological effects thereto. Examples of such salts are (a) salts formed with cations such as sodium, potassium, NH₄⁺, magnesium, calcium, polyamines such as spermine and spermidine, etc.; (b) acid addition salts formed with inorganic acids, for example, hydrochloric acid, hydrobromic acid, sulfuric acid, phosphoric acid, nitric acid and the like; (c) salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, napthalenesulfonic acid, methanesulfonic acid, p-toluenesulfonic acid, napthalenedisulfonic acid, polygalacturonic acid, and the like; and (d) salts formed from elemental anions such as chlorine, bromine, and iodine.

The present invention provides for the use of SSOs having the characteristics set forth above for the preparation of a medicament for increasing the ratio of a soluble form of a TNFR superfamily member to its corresponding membrane bound form, in a patient afflicted with an inflammatory disorder involving excessive activity of a cytokine, such as TNF-α, as discussed above. In the manufacture of a medicament according to the invention, the SSOs are typically admixed with, inter alia, an acceptable carrier. The carrier must, of course, be acceptable in the sense of being compatible with any other ingredients in the formulation and must not be deleterious to the patient. The carrier may be a solid or liquid. SSOs are incorporated in the formulations of the invention, which may be prepared by any of the well known techniques of pharmacy consisting essentially of admixing the components, optionally including one or more accessory therapeutic ingredients.

Formulations of the present invention may comprise sterile aqueous and non-aqueous injection solutions of the active compounds, which preparations are preferably isotonic with the blood of the intended recipient and essentially pyrogen free. These preparations may contain anti-oxidants, buffers, bacteriostats, and solutes which render the formulation isotonic with the blood of the intended recipient. Aqueous and non-aqueous sterile suspensions can include, but are not limited to, suspending agents and thickening agents. The formulations may be presented in unit dose or multi-dose containers, for example, sealed ampoules and vials, and may be stored in freeze-dried (lyophilized) condition requiring only the addition of the sterile liquid carrier, for example, saline or water-for-injection immediately prior to use.

In the formulation the SSOs may be contained within a lipid particle or vesicle, such as a liposome or microcrystal, which may be suitable for parenteral administration. The particles may be of any suitable structure, such as unilamellar or plurilameller, so long as the SSOs are contained therein. Positively charged lipids such as N-[1-(2,3-dioleoyloxi)propyl]-N,N,N-trimethyl-ammoniummethylsulfate, or "DOTAP," are particularly preferred for such particles and vesicles. The preparation of such lipid particles is well known. [See references in U.S. Pat. 5,976,879 col. 6]

The SSO can be targeted to any element or combination of elements that regulate splicing, including the 3'splice site, the 5' splice site, the branch point, the polypyrimidine tract, exonic splicing ehancers, exonic splicing silencers, intronic splicing enhancers, and intronic splicing silencers. The determination of the sequence of the SSO can be guided by the following tables that shows the activities of the SSOs whose sequences and locations are found as depicted in Figures **4****,** **5****,** and **8****.** The person skilled in the art will note that: 1) SSOs complementary to the exon need not be complementary to either the splice acceptor or splice donor sites, note SSOs A7-10, B7-7 and B7-9, Table 1; 2) SSOs complementary to sequences of the intron and as few as one nucleotide of the exon can be operative, note A8-5 and B7-6, Table 1; 3) SSOs complementary to the intron immediately adjacent to the exon can also be effective, note 3312, Table 2; and 4) efficacy of an oligonucleotide alone is usually predictive of the efficacy of the SSO in combination with other SSOs.

Those skilled in the art can appreciate that the invention as directed toward human TNF-α receptors can be practiced using SSO having a sequence that is complementary to at least 10, preferably between 15 and 20 nucleotides of the portions of the TNFR1 or TNFR2 genes comprising exons 7 or 8 and their adjacent introns. It is further preferred that at least one nucleotide of the exon itself is included within the complementary sequence. SEQ ID Nos: 1-4 contain the sequence of Exons 7 and 8 of the TNFR1 (SEQ ID Nos: 1 and 2) and TNFR2 (SEQ ID Nos; 3 and 4) and 50 adjacent nucleotides of the flanking introns. When affinity-enhancing modifications are used, including but not limited to LNA or G-clamp nucleotides, the skilled person recognizes the length of the SSO can be correspondingly reduced. When alternating conventional and LNA nucleotides are used a length of 16 is effective. The pattern of alternation of LNA and conventional nucleotides is not important.

Those skilled in the art will also recognize that the selection of SSO sequences must be made with care to avoid self-complementary SSO, which may lead to the formation of partial "hairpin" duplex structures. In addition, high GC content should be avoided to minimize the possibility of non-specific base pairing. Furthermore, SSOs matching off-target genes, as revealed for example by BLAST, should also be avoided.

In some situations, it may be preferred to select an SSO sequence that can target a human and at least one other species. These SSOs can be used to test and to optimize the invention in said other species before being used in humans, thereby being useful for regulatory approval and drug development purposes. For example, SEQ ID Nos: 74, 75, 77, 78, 80, and 89, which target human TNFR2 are also 100% complementary to the corresponding Macaca Mullata sequences. As a result these sequences can be used to test treatments in monkeys, before being used in humans.

It will be appreciated by those skilled in the art that various omissions, additions and modifications may be made to the invention described above without departing from the scope of the invention, and all such modifications and changes are intended to fall within the scope of the invention, as defined by the appended claims. All references, patents, patent applications or other documents cited are herein incorporated by reference.

### Example 1

### Materials and Methods

*Oligonucleotides.* All uniformly modified 2'-O-methyl-ribonucleoside-phosphorothioate (2'-OMe) 20-mers were synthesized by Trilink Biotechnologies, San Diego, CA. Their sequences are listed in Table 1. Tables 2 and 3 show the sequences of chimeric LNA SSOs with alternating 2'deoxy- and 2'O-4'-(methylene)-bicyclic-ribonucleoside phosphorothioates. These were synthesized by Santaris Pharma, Denmark. For each LNA oligonucleotide, the 5'-terminal nucleoside was a 2'O-4'-methylene-ribonucleoside and the 3'-terminal ribonucleoside was a 2'deoxy-ribonucleoside.

*Cell culture and transfections.* NIH-3T3 cells were maintained (37°C, 5% CO₂) in Dulbecco's modified Eagle's media (DMEM) supplemented with 10% Colorado fetal calf serum and antibiotic. L929 cells were maintained (37°C, 5% CO₂) in minimal essential media supplemented with 10% fetal bovine serum and antibiotic. For transfection, either NIH-3T3 or L929 cells were seeded in 24-well plates at 10⁵ cells per well and transfected 24 hours later. Oligonucleotides were complexed, at the indicated concentrations, with 2 µL of Lipofectamine™ 2000 transfection reagent (Invitrogen) as per the manufacturer's directions. The nucleotide/lipid complexes were then applied to the cells and incubated for hours. The media was then aspirated and cells harvested with TRI-Reagent™ (MRC, Cincinnati, OH).

*RT-PCR.* Total RNA was isolated with TRI-Reagent (MRC, Cincinnati, OH) and TNFR1 or TNFR2 mRNA was amplified by RT-PCR using r*Tth* polymerase (Applied Biosystems) following supplier directions. Murine TNFR1 mRNA was amplified using forward primer PS009 (SEQ ID No: 111) (5'- GAA AGT GAG TGC GTC CCT TGC-3') and reverse primer PS010 (SEQ ID No: 112) (5'- GCA CGG AGC AGA GTG ATT CG-3'). Murine TNFR2 mRNA was amplified using forward primer PS003 (SEQ ID No: 113) (5'-GAG CCC CAA ATG GAA ATG TGC-3') and reverse primer PS004 (SEQ ID No: 114) (5'-GCT CAA GGC CTA CTG CC-3'). Human TNFR2 mRNA was amplified using forward primer (SEQ ID No: 115) (5'-ACT GAA ACA TCA GAC GTG GTG TGC-3') and reverse primer (SEQ ID No: 116) (5'-CCT TAT CGG CAG GCA AGT GAG-3'). A Cy5-labeled dCTP (GE Healthcare) was included in the PCR step for visualization (0.1 µL per 50 µL PCR reaction). Cycles of PCR proceeded: 95°C, 60 sec; 56°C, 30 sec; 72°C, 60 sec for 22 cycles total. The PCR products were separated on a 10% non-denaturing polyacrylamide gel, and Cy5-labeled bands were visualized with a Typhoon™ 9400 Scanner (GE Healthcare). Scans were quantified with ImageQuant™ (GE Healthcare) software.

*Mouse hepatocyte cultures.* For hepatocyte collection, livers of mice were perfused with RPMI medium containing 0.53 mg/ml of collagenase (Worthington Type 1, code CLS). After perfusion, the cell suspension was collected and seeded in a stop solution of RPMI with 10% (vol/vol) FBS and 0.5% penicillin-streptomycin plus 1 nM insulin and 13 nM dexamethasone. Approximately 3 × 10⁵ cells were seeded on a six-well collagen-coated plate. The seeding medium was replaced 1 hour later with maintenance medium consisting of seeding medium without the 10% (vol/vol) FBS. Varying amounts of oligonucleotide-lipid complexes were applied 24 hours later. Cells were lysed 24 hours after transfection with TRI-Reagent™.

*Human hepatocyte cultures.* Human hepatocytes were obtained in suspension either from ADMET technologies, or from The UNC Cellular Metabolism and Transport Core at UNC-Chapel Hill. Cells were washed and suspended in RPMI 1640 supplemented with 10% FBS, 1 µg/mL human insulin, and 13 nM Dexamethasone. Hepatocytes were plated in 6-well plates at 0.5 × 10⁶ cells per plate in 3 mL media. After 1-1.5 hours, non-adherent cells were removed, and the media was replaced with RPMI 1640 without FBS, supplemented with 1 µg/mL human insulin, and 130 nM Dexamethasone.

For delivery of LNA SSOs to hepatocytes in 6-well plates, 10 µL of a 5 µM LNA stock was diluted into 100 µL of OPTI-MEM™, and 4 µL of Lipofectamine™ 2000 was diluted into 100 µL of OPTI-MEM™. The 200 µL complex solution was then applied to the cells in the 6-well plate containing 2800 µL of media, for a total of 3000 µL. The final LNA concentration was 17 nM. After 24 hours, cells were harvested in TRI-Reagent™. Total RNA was isolated per the manufacturers directions. Approximately 200 ng of total RNA was subjected to reverse transcription-PCR (RT-PCR).

*ELISA.* To determine the levels of soluble TNFR2 in cell culture media or mouse sera, the Quantikine® Mouse sTNF RII ELISA kit from R&D Systems (Minneapolis, MN) was used. To determine the levels of soluble TNFR1 in cell culture media or mouse sera, the Quantikine® Mouse sTNF RI ELISA kit from R&D Systems (Minneapolis, MN) was used. Note, the antibodies used for detection also detect the protease cleavage forms of the receptor.

For cell culture studies, extracellular media was collected at 72 hours post transfection. The assay was performed according to the manufacturer's guide, using 50 µL of undiluted media. The assay readings were performed using a microplate reader set at 450 nm, with wavelength correction set at 570 nm.

For mouse *in vivo* studies, blood from the animals was clotted for 1 hour at 37°C and centrifuged for 10 min at 14,000 rpm (Jouan BRA4i centrifuge). Sera was collected and assayed according to the manufacturer's guide, using 50 µL of mouse sera, diluted 1:10. The assay readings were performed using a microplate reader set at 450 nm, with wavelength correction set at 570 nm.

*L929 cytotoxicity assay.* L929 cells plated in 96-well plates at 10⁴ cells per plate were treated with 0.1 ng/mL TNF-α (TNF) and actinomycin D (ActD) in the presence of 10% serum from mice treated with the indicated oligonucleotide in 100 µL total cell culture media. Control lanes were plated in 10% serum from untreated mice. 24 hours later, cell viability was measured by adding 20 µL CellTiter 96® Aqueous Solution (Promega) and measuring absorbance at 490 nm with a microplate reader. Cell viability was normalized to cells untreated with TNF/ActD.

### Example 2

### Testing of SSOs for Splice Switching Activity

SSOs were synthesized, transfected into either NIH-3T3 or L929 cells. Total RNA from the cells was analyzed by RT-PCR to assess the splice switching ability of the SSO. Table 1 contains the sequences and the splice switching activities of 20 nucleotide 2'O-Me-ribonucleoside-phosphorothioate murine SSOs. Table 2 contains the sequences and the splice switching activities of 16 nucleotide chimeric LNA murine SSOs. Table 3 contains the sequences and the splice switching activities of 16 nucleotide chimeric LNA human SSOs. Each table also lists the target site for each SSO by complementary regions and number of nucleotides; e.g., 16:E7(8:8) means complementary to the 3'-most 8 nucleotides ofintron 6 and the 5'-most 8 nucleotides of exon 7; E7(16) means complementary to 16 nucleotides in exon 7; and E8:I8(7:9) means complementary to the 3'-most 7 nucleotides of exon 8 and the 5'-most 9 nucleotides of intron 8.

**Table 1**

| **2'O-Me-ribonucleoside-phosphorothioate mouse targeted SSO** | | | | |
|---|---|---|---|---|
| **SEQ ID.** | **Name*** | **Sequence (5'-3')** | **' Activity** | **Target Site** |
| 5 | A7-1 | CCG CAG UAC CUG CAG ACC AG | - | I6:E7(6:14) |
| 6 | A7-2 | GUA CCU GCA GAC CAG AGA GG | - | I6:E7(13:7) |
| 7 | A7-3 | CUG CAG ACC AGA GAG GUU GC | - | I6:E7(18:2) |
| 8 | A7-4 | ACU GAU GGA GUA GAC UUC GG | + | E7:I7(18:2) |
| 9 | A7-5 | AGU CCU ACU UAC UGA UGG AG | + | E7:I7(8:12) |
| 10 | A7-6 | CCA AAG UCC UAC UUA CUG AU | - | E7:I7(1:19) |
| 11 | A7-7 | AGA UAA CCA GGG GCA ACA GC | - | E7 (20) |
| 12 | A7-8 | AGG AUA GAA GGC AAA GAC CU | - | E7(20) |
| 13 | A7-9 | GGC ACA UUA AAC UGA UGA AG | - | E7 (20) |
| 14 | A7-10 | GGC CUC CAC CGG GGA UAU CG | + | E7 (20) |
| 15 | A8-1 | CUG GAG AAC AAA GAA ACA AG | - | I7:E8(19:1) |
| 16 | A8-2 | AUC CCU ACA AAC UGG AGA AC | - | I7:E8(8:12) |
| 17 | A8-3 | GGC ACG GGA UCC CUA CAA AC | ++ | E8 (20) |
| 18 | A8-4 | CUU CUC ACC UCU UUG ACA GG | ++ | E8:I8(12:8) |
| 19 | A8-5 | UGG AGU CGU CCC UUC UCA CC | + | E8:18(1:19) |
| 20 | B7-1 | CUC CAA CAA UCA GAC CUA GG | +++ | I6:E7(5:15) |
| 21 | B7-2 | CAA UCA GAC CUA GGA AAA CG | + | I6:E7(11:9) |
| 22 | B7-3 | AGA CCU AGG AAA ACG GCA GG | - | I6:E7(16:4) |
| 23 | B7-4 | CCU UAC UUU UCC UCU GCA CC | - | E7:I8(14:6) |
| 24 | B7-5 | GAG CAG AAC CUU ACU UUU CC | ++ | E7:I8(6:14) |
| 25 | B7-6 | GAC GAG AGC AGA ACC UUA CU | ++ | E7:17(1:19) |
| 26 | B7-7 | UCA GCA GAC CCA GUG AUG UC | ++ | E7(20) |
| 27 | B7-8 | AUG AUG CAG UUC ACC AGU CC | + | E7(20) |
| 28 | B7-9 | UCA CCA GUC CUA ACA UCA GC | ++ | E7(20) |
| 29 | B7-10 | CCU CUG CAC CAG GAU GAU GC | ++ | E7(20) |
| 30 | B8-1 | UUC UCU ACA AUG AAG AGA GG | - | I7:E8(16:4) |
| 31 | B8-2 | GGC UUC UCU ACA AUG AAG AG | - | I7:E8(13:7) |
| 32 | B8-3 | UGU AGG CAG GAG GGC UUC UC | ++ | I7:E8(1:19) |
| 33 | B8-4 | ACU CAC CAC CUU GGC AUC UC | ++ | E8:I8(14:6) |
| 34 | B8-5 | GCA GAG GGA UAC UCA CCA CC | - | E8:I8(4:16) |

| | | | | |
|---|---|---|---|---|
| *SSOs with the prefix "A" are directed to TNFR1 and with "B" to TNFR2. | | | | |

**Table 2**

| **LNA-2'deoxy-ribonucleosidephosphorothioate chimeric mouse targeted SSO** | | | | |
|---|---|---|---|---|
| **SEQ ID.** | **Name** | **Sequence 5' to 3'** | **Activity** | **Target Site** |
| | | **TNFR2 Exon 7** | | |
| 35 | 3272 | CAA TCA GAC CTA GGA A | - | I6:E7(7:9) |
| 36 | 3303 | CAA CAA TCA GAC CTA G | - | I6:E7(4:12) |
| 37 | 3304 | CAG ACC TAG GAA AAC G | - | I6:E7(11:5) |
| 38 | 3305 | AGC AGA CCC AGT GAT G | ++ | E7 (16) |
| 39 | 3306 | CCA GTC CTA ACA TCA G | + | E7 (16) |
| 40 | 3307 | CAC CAG TCC TAA CAT C | + | E7(16) |
| 41 | 3308 | CTG CAC CAG GAT GAT G | + | E7(16) |
| 42 | 3309 | ACT TTT CCT CTG CAC C | + | E7:I7(14:2) |
| 43 | 3310 | CCT TAC TTT TCC TCT G | - | E7:17(8:8) |
| 44 | 3311 | CAG AAC CTT ACT TTT C | ++ | E7:I7(5:11) |
| 45 | 3274 | AGA GCA GAA CCT TAC T | ++ | E7:I7(1:15) |
| 46 | 3312 | GAG AGC AGA ACC TTA C | ++ | E7:I7(0:16) |
| 47 | 3273 | ACC TTA CTT TTC CTC T | - | E7:17(9:7) |

| | | **TNFR2 Exon 8** | | |
|---|---|---|---|---|
| 48 | 3313 | CTT CTC TAC AAT GAA G | - | I7:E8(11:5) |
| 49 | 3314 | CCT TGG CAT CTC TTT G | - | E8(16) |
| 50 | 3315 | TCA CCA CCT TGG CAT C | + | E8:I8(12:4) |
| 51 | 3316 | ACT CAC CAC CTT GGC A | + | E8:I8(10:6) |
| 52 | 3317 | GAT ACT CAC CAC CTT G | + | E8:I8(7:9) |
| 53 | 3631 | CTA CAA TGA AGA GAG G | - | 17(16) |
| 54 | 3632 | CTC TAC AAT GAA GAG A | - | I7;E8(14:2) |
| 55 | 3633 | AGG GAT ACT CAC CAC C | + | E8:I8(4:12) |
| 56 | 3634 | CAG AGG GAT ACT CAC C | + | E8:I8(1:15) |
| 57 | 3635 | CGC AGA GGG ATA CTC A | + | I8(16) |
| 58 | 3636 | GAA CAA GTC AGA GGC A | - | 17(16) |
| 59 | 3637 | GAG GCA GGA CTT CTT C | - | 17(16) |

| | | **TNFR1 Exon 7** | | |
|---|---|---|---|---|
| 60 | 3325 | CGC AGT ACC TGC AGA C | + | I6:E7(8:8) |
| 61 | 3326 | AGT ACC TGC AGA CCA G | - | I6:E7(11:5) |
| 62 | 3327 | GGC AAC AGC ACC GCA G | - | E7(16) |
| 63 | 3328 | CTA GCA AGA TAA CCA G | - | E7(16) |
| 64 | 3329 | GCA CAT TAA ACT GAT G | - | E7(16) |
| 65 | 3330 | CTT CGG GCC TCC ACC G | - | E7(16) |
| 66 | 3331 | CTT ACT GAT GGA GTA G | - | E7:I7(11:5) |
| 67 | 3332 | CCT ACT TAC TGA TGG A | - | E7:I7(7:9) |
| 68 | 3333 | GTC CTA CTT ACT GAT G | + | E7:I7(5:11) |

| | | **TNFR1 Exon 8** | | |
|---|---|---|---|---|
| 69 | 3334 | TCC CTA CAA ACT GGA G | + | E7:I7(5:11) |
| 70 | 3335 | GGC ACG GGA TCC CTA C | + | E8(16) |
| 71 | 3336 | CTC TTT GAC AGG CAC G | + | E8(16) |
| 72 | 3337 | CTC ACC TCT TTG ACA G | - | E8:I8(11:5) |
| 73 | 3338 | CCT TCT CAC CTC TTT G | - | E8:I8(7:9) |

**Table 3:**

| **LNA-2'deoxy-ribonucleosidephosphorothioate chimeric human targeted SSO** | | | | |
|---|---|---|---|---|
| **SEQ ID.** | **Name** | **Sequence 5' to 3'** | **Activity '** | **Target Site** |
| | | **TNFR2 Exon 7** | | |
| 74 | 3378 | CCA CAA TCA GTC CTA G | ++ | I6:E7(4:12) |
| 75 | 3379 | CAG TCC TAG AAA GAA A | ++ | I6:E7(11:5) |
| 76 | 3380 | AGT AGA CCC AAG GCT G | - | E7(16) |
| 77 | 3381 | CCA CTC CTA TTA TTA G | + | E7 (16) |
| 78 | 3382 | CAC CAC TCC TAT TAT T | + | E7(16) |
| 79 | 3383 | CTG GGT CAT GAT GAC A | - | E7 (16) |
| 80 | 3384 | ACT TTT CAC CTG GGT C | ++ | E7:I7(14:2) |
| 81 | 3385 | TCT TAC TTT TCA CCT G | - | E7:I7(10:6) |
| 82 | 3459 | TGG ACT CTT ACT TTT C | ++ | E7;I7(5:11) |
| 83 | 3460 | AGG ATG GAC TCT TAC T | - | E7:I7(1:15) |
| 84 | 3461 | AAG GAT GGA CTC TTA C | + | I7(16) |

| | | **TNFR2 Exon 8** | | |
|---|---|---|---|---|
| 85 | 3462 | CTT CTC TAT AAA GAG G | - | I7:E8(11:5) |
| 86 | 3463 | CCT TGG CTT CTC TCT G | + | E8(16) |
| 87 | 3464 | TCA CCA CCT TGG CTT C | + | E8:I8(12:4) |
| 88 | 3465 | ACT CAC CAC CTT GGC T | + | E8:I8(10:6) |
| 89 | 3466 | GAC ACT CAC CAC CTT G | + | E8:I8(7:9) |

| | | **TNFR1 Exon 7** | | |
|---|---|---|---|---|
| 90 | 3478 | TGT GGT GCC TGC AGA C | N/A | I6:E7(8:8) |
| 91 | 3479 | GGT GCC TGC AGA CAA A | N/A | I6:E7(11:5) |
| 92 | 3480 | GGC AAC AGC ACT GTG G | N/A | E7(16) |
| 93 | 3481 | CAA AGA AAA TGA CCA G | N/A | E7(16) |
| 94 | 3482 | ATA CAT TAA ACC AAT G | N/A | E7(16) |
| 95 | 3483 | GCT TGG ACT TCC ACC G | N/A | E7(16) |
| 96 | 3484 | CTC ACC AAT GGA GTA G | N/A | E7:17(11:5) |
| 97 | 3485 | CAC TCA CCA ATG GAG T | N/A | E7:I7(9:7) |
| 98 | 3587 | CCC ACT CAC CAA TGG A | N/A | E7:I7(7:9) |
| 99 | 3588 | CCC CCA CTC ACC AAT G | N/A | E7:I7(5:11) |
| 100 | 3589 | AAA GCC CCC ACT CAC C | N/A | E7:I7(1:15) |

| | | **TNFR1 Exon 8** | | |
|---|---|---|---|---|
| 101 | 3590 | TTT CCC ACA AAC TGA G | N/A | I7:E8(5:11) |
| 102 | 3591 | GGT GTC GAT TTC CCA C | N/A | E8(16) |
| 103 | 3592 | CTC TTT TTC AGG TGT C | N/A | E8(16) |
| 104 | 3593 | CTC ACC TCT TTT TCA G | N/A | E8:I8(11:5) |
| 105 | 3594 | TCA TCT CAC CTC TTT T | N/A | E8:I8(7:9) |

| | | **Control** | | |
|---|---|---|---|---|
| 106 | 3083 | GCT ATT ACC TTA ACC C | N/A | N/A |

### Example 3

### Effect of SSOs on L929 Mouse Cells

Single LNA SSOs were transfected into L929 murine cells and analyzed for splice switching of TNFR2. FIG. 9 (top) shows the splice switching results of LNAs targeted towards mouse exon 7. Of the LNAs tested, at least 9 showed some activity. In particular, LNA 3312, 3274 and 3305 induced skipping of exon 7 to 50% or greater; LNA 3305 treatment resulted in almost complete skipping. FIG. 9 (bottom) shows the activity of SSOs targeted towards mouse exon 8. The data indicate that LNA 3315 and 3316 are equally potent at inducing an appromixately 20% skipping of exon 8. Note that exon 8 is small (35 nts), and therefore the difference in exon 8-containing and exon 8-lacking PCR fragments is also small.

### Example 4

### Effect of Multiple SSOs on L929 Mouse Cells

LNA SSOs targeting exon 7 and 8 were transfected in combination into L929 cells to determine whether such treatment would result in generation of TNFR2 Δ7/8 mRNA. The data in FIG. 10 show that the combination of exon 8 targeted 3315 or 3316 with one of exon 7 targeted LNA 3305, 3309, 3312, or 3274 induced skipping of both exons simultaneously. In particular, the combination of LNAs 3305 and 3315 resulted in greater than 60% shift to the A7/8 mRNA, with the remainder being almost entirely Δ7 mRNA. Other combinations were also effective; 3274 with 3315 led to a 50% shift to the Δ7/8 mRNA. These data indicate that LNA SSOs are very effective at inducing alternatively spliced TNFR2 mRNAs. Similarly, combinations of LNA SSOs targeted to TNFR1 exon 7 and 8 also induced shifting of their respective exons in L929 cells (FIG. 11).

### Example 5

### Effect of LNA SSOs on Primary Mouse Hepatocytes

The TNFR2 LNA SSOs were transfected into primary mouse hepatocytes, and were found to be equally effective in splice switching in these cells. In particular, treatment with LNA 3274 or 3305 in combination with LNA 3315 showed splice shifting profiles very similar to those found in L929 cells (FIG. 12). These data confirm splice shifting occurs in intended *in vivo* cellular targets.

### Example 6

### Secretion of TNFR2 Splice Variants from Murine Cells

The ability of LNA SSOs to induce soluble TNFR2 protein production and secretion into the extracellular media was tested. L929 cells were treated with the LNA SSOs as above, and extracellular media samples were collected 48 hours after transfection. The samples were quantified by an ELISA specific for soluble TNFR2 (for either Δ7 and Δ7/8 protein isoforms). The FIG. 13 left panel indicates that the LNAs that best induced shifts in RNA splicing, also secreted the most protein into the extracellular media. In particular, LNAs 3305, 3312 and 3274 performed best, increasing soluble TNFR2 at least 3.5-fold over background, and yielding 250 pg/mL soluble splice variant. Increases were also seen in similarly treated primary mouse hepatocytes (FIG. 13, right panel). In these primary cells, treatment with LNA 3274 or 3305 alone gave approximately 2.5-fold increases in soluble TNFR2 in the extracellular media, yielding ∼200 pg/mL of the soluble splice variant, and the combination of 3274 or 3305 with 3315 also increased protein production. Consequently, induction of the splice variant mRNA correlated with production and secretion of the soluble TNFR2.

### Example 7

### Effect of LNA SSOs on Primary Human Hepatocytes

LNA SSOs for human TNFR2 pre-mRNA were transfected into cultured primary human hepatocytes. FIG. **14** shows that 7 of 10 SSOs targeted to exon 7 exhibited some splice switching activity. In particular, LNAs 3378, 3384 and 3479 showed at least 75% skipping of exon 7. Likewise, 4 of the 5 exon 8 targeted SSOs showed activity. Interestingly, LNAs 3464, 3465, or 3466 alone was sufficient to induce Δ7/8 splice removal, an observation not seen in mouse cells. Hence, only one SSO may be required to induce skipping of both exon 7 and exon 8. These data confirm splice shifting occurs in intended human therapeutic targets.

### Example 8

### In Vivo Injection of LNA SSOs in Mice

LNA 3305, at doses from 3 mg/kg to 25 mg/kg diluted in saline only, were injected intraperitoneal (i.p.) once a day for 4 days into mice. The mice were sacrificed on day 5 and total RNA from the liver was analyzed by RT-PCR. The data show splice switching efficacy similar to that found in cell culture. At the maximum dose of 25mg/kg, LNA 3305 induced almost full conversion to Δ7 mRNA (FIG. **15****,** bottom panel).

A similar procedure using LNA 3274 induced about 20% conversion to Δ7 mRNA. To optimize the induction of Δ7 mRNA LNA 3274, both the dose regimen and time between the last injection, and sacrifice of the animals was varied. LNA 3274, at 25 mg/kg diluted in saline only, were injected (i.p.) once a day for 4 days into mice. In mice analyzed on day 15, whereas those analyzed on day five demonstrated only a 20% shift to Δ7 mRNA (FIG. **15****,** top panel). Furthermore, mice given injections for 10 days, and sacrificed on day 11 showed a 50% induction of Δ7 mRNA (FIG. **15** top). These *in vivo* data suggest that TNFR2 LNA SSOs can persist in the liver and induce splice switching for at least 10 days after administration.

### Example 9

### Circulatory TNFR Splice Variants

Induction of the Δ7 mRNA in liver should produce soluble TNFR, which can be secreted and accumulate in the circulation. Accordingly, mice were treated with LNA 3274, 3305, or the control 3083 alone i.p. at 25mg/kg/day for 10 days. Mice were bled before injection and again 1, 5 and 10 days after the last injection. Serum was quantified for concentration of soluble TNFR2. FIG.**16** shows that LNA treatment induced 6000-8000 pg/mL of soluble TNFR2 (Δ7), which was significantly over background for at least 10 days.

The same samples were assayed for production of soluble TNFR1. No increase in soluble TNFR1 was observed (FIG. **17****).**

To test the effects at longer time points, the same experiment was carried out, and mice were analyzed for soluble TNFR2 in the serum up to 27 days after the last injection. The results show only a slight decrease in soluble TNFR2 levels 27 days after the last LNA SSO injection (FIG. **18****).** This data suggests that the effects of the LNAs persist for at least 27 days.

### Example 10

### Measurement of Anti-TNF-α Activity of Mice Treated with LNA SSOs

The anti-TNF-α activity of serum from LNA 3274 treated mice was tested in an L929 cytotoxicity assay. In this assay, serum is tested for its ability to protect cultured L929 cells from the cytotoxic effects of a fixed concentration of TNF-α. L929 cells were seeded in 96-well plates at 2 x 10⁴ cells per well in 100 µL of complete MEM media (containing 10% regular FBS) and allowed to grow for 24 hours at 37°C. As shown in FIG. **19****,** serum from mice treated with LNA 3274 but not control LNAs (3083 or 3272) increased viability of the L929 cells exposed to 0.1 ng/mL TNF-α. Hence, the LNA 3274 serum contained Δ7 TNFR2 TNF-α antagonist, sufficient to bind and inactivate TNF-α, and thereby protect the cells from the cytotoxic effects of TNF-α. This anti-TNF-a activity was present in the serum of animals 5 and 27 days after the last injection of the 3274 LNA.

### Example 11

### Comparison of LNA SSOs to other anti-TNF-α agents

L929 cells were seeded as in Example 10. Samples were prepared containing 90 µL of serum-free MEM, 0.1 ng/ml TNF-α (TNFR) and 1 µg/ml of actinomycin D (ActD), with either (i) rsTNFR2 (recombinant soluble) (0.01-3 µg/mL), (ii) serum from LNA 3274 treated mice (1.25-10%, diluted in serum from untreated mice) or (iii) Enbrel® (0.45-150 pg/ml) to a final volume of 100 µl with a final mouse serum concentration of 10%. The samples were incubated at room temperature for 30 minutes. Subsequently, the samples were applied to the plated cells and incubated for ∼24 hours at 37°C in a 5% CO₂ humidified atmosphere. Cell viability was measured by adding 20 µL CellTiter 96® Aqueous Solution (Promega) and measuring absorbance at 490 nm with a microplate reader. Cell viability, as shown in FIG. **20****,** was normalized to cells untreated with TNF/ActD.

The invention also relates to the following embodiments numbered as 1-72 (referred to as claims):
1. A method of treating an inflammatory disease or condition which comprises administering one or more splice switching oligomers (SSOs) to a subject for a time and in an amount to reduce the activity of a ligand for a receptor of the tumor necrosis factor receptor (TNFR) superfamily, wherein said one or more SSOs are capable of altering the splicing of a pre-mRNA encoding said receptor to increase production of a stable, secreted, ligand-binding form of said receptor.
2. The method of claim 1, wherein production of a membrane bound form of said receptor is decreased.
3. The method of either claim **1** or **2**, wherein said receptor is a mammalian receptor selected from the group consisting of TNFRSF1A, TNFRSF1B, TNFRSF3, TNFRSF5, TNFRSF8, and TNFRSF11A.
4. The method of claim **3,** wherein said mammalian receptor is a human receptor, a primate receptor, or a murine receptor.
5. The method of claim **4,** wherein said receptor is TNFRSF1A, or TNFRSF1B
6. The method of claim **5,** wherein said receptor is a human TNFRSF1A or a human TNFRSF1B.
7. The method of claim **6,** wherein said receptor is a human TNFRSF1B.
8. The method of either claim **1** or **3,** wherein said ligand is TNF-α, RANKL, CD40L, LT-α, or LT-β.
9. The method of claim **8,** wherein said ligand is TNF-α.
10. The method of claim **1,** wherein said disease or condition is selected from the group consisting of rheumatoid arthritis, juvenile rheumatoid arthritis, psoriasis, psoriatic arthritis, ankylosing spondylitis, inflammatory bowel disease (Crohn's disease or ulcerative colitis), hepatitis, sepsis, alcoholic liver disease, and non-alcoholic steatosis.
11. The method of claim **10,** wherein said hepatitis involves or is hepatitis associated with hepatitis A virus, hepatitis B virus, hepatitis C virus, or ischemia/reperfusion.
12. The method of any one of claims **1-11,** wherein two or more SSOs are administered.
13. The method of any one of claims **1-12,** wherein said altering the splicing of said pre-mRNA comprises excising one or more exons encoding at least part of the membrane spanning domain of said receptor.
14. The method of claim **1,** wherein said receptor is TNFRSF1A, TNFRSF1B, TNFRSF3, TNFRSF5, or TNFRSF11A and said altering the splicing of said pre-mRNA comprises excising exon 7, exon 8, or both from said pre-mRNA.
15. The method of claim **14,**wherein said altering the splicing of said pre-mRNA comprises excising exon 7.
16. The method of either claim **14** or **15,** wherein said receptor is TNFRSF1A or TNFRSF1B.
17. The method of claim **1,** wherein said receptor is TNFRSF8 and said altering the splicing of said pre-mRNA comprises excising exon 11 from said pre-mRNA.
18. The method of claim **14,** wherein said SSO comprises from at least 10 to at least 20 nucleotides which are complementary to a contiguous sequence from SEQ ID Nos: 1, 2, 3 or 4.
19. The method of claim **18,** wherein said nucleotides are complementary to one or more nucleotides from an exon encoded by nucleotides 51-164 of SEQ ID No: 1, 51-79 of SEQ ID No: 2, 51-127 of SEQ ID No: 3, or 51-85 of SEQ ID No: 4.
20. The method of claim **19,** wherein said nucleotides are complementary to at least 2, 5, 8 or 10 nucleotides of one of said exons.
21. The method of claim **19,** wherein said nucleotides are complementary only to sequences selected from said exons.
22. The method of claim **18,** wherein said SSOs are complementary only to an intron encoded by nucleotides 1-50 of SEQ ID No: 1, 165-215 of SEQ ID No: 1, 1-50 of SEQ ID No: 2, 80-130 of SEQ ID No: 2,1-50 of SEQ ID No: 3,128-178 of SEQ ID No: 3, nucleotides 1-50 of SEQ ID No: 4, or 86-136 of SEQ ID No: 4.
23. The method of any one of claims **1-22,** wherein said SSOs comprise one or more nucleotides or nucleosides independently selected from the group consisting of 2'-deoxyribonucleotides, 2'O-Me ribonucleotides, 2'O-MOE ribonucleotides, hexitol (HNA) nucleotides or nucleosides, 2'O-4'C-linked bicyclic ribofuranosyl (LNA) nucleotides or nucleosides, phosphorothioate analogs of any of the foregoing, peptide nucleic acid (PNA) analogs of any of the foregoing; methylphosponate analogs of any of the foregoing, peptide nucleic acid analogs of any of the foregoing, N3'→P5' phosphoramidate analogs of any of the foregoing, and phosphorodiamidate morpholino nucleotide analogs of any of the foregoing, and combinations thereof.
24. the method of claim **23,** wherein said SSOs comprise only nucleotides or nucleosides independently selected from the group consisting of 2'-deoxyribonucleotides, 2'O-Me ribonucleotides, 2'O-MOE ribonucleotides, hexitol (HNA) nucleotides or nucleosides, 2'O-4'C-linked bicyclic ribofuranosyl (LNA) nucleotides or nucleosides, phosphorothioate analogs of any of the foregoing, peptide nucleic acid (PNA) analogs of any of the foregoing; methylphosponate analogs of any of the foregoing, peptide nucleic acid analogs of any of the foregoing, N3'→P5' phosphoramidate analogs of any of the foregoing, and phosphorodiamidate morpholino nucleotide analogs of any of the foregoing, and combinations thereof.
25. The method of claim **23,** wherein said 2'O-4'C-linked bicyclic ribofuranosyl (LNA) nucleotides or nucleosides are 2'O-4'C-(methylene)-ribofuranosyl nucleotides or nucleosides, respectively, or 2'O-4'C-(ethylene)-ribofuranosyl nucleotides or nucleosides, respectively.
26. The method of claim **23,** wherein said SSOs comprise one or more nucleotides or nucleosides independently selected from the group consisting af 2'O-Me ribonucleotides and 2'0-4'C-linked bicyclic ribofuranosyl (LNA) nucleotides or nucleosides.
27. The method of claim **23,** wherein said SSOs contains at least four LNA nucleotides or nucleosides.
28. The method of claim **18** wherein the sequence of said SSO comprises a sequence selected from the group consisting of SEQ ID Nos: 74, 75, 77, 78, 80, 82, 84, and 86-89.
29. The method of any one of claims **1-28,** wherein said administration is parenteral, topical, oral, rectal, or pulmonary.
30. The method of claim **29,** wherein said parenteral administration is by intraperitoneal, intravenous, intraarterial, subcutaneous, or intramuscular injection or infusion.
31. The method of claim **29,** wherein said administration is oral or subcutaneous.
32. A method of increasing the production of a stable, secreted, ligand-binding form of a receptor from the TNFR superfamily in a cell, which comprises administering one or more splice switching oligomers (SSOs) to said cell, wherein said one or more SSOs are capable of altering the splicing of a pre-mRNA encoding said receptor to increase production of a stable, secreted, ligand-binding form of said receptor.
33. The method of claim **32,** wherein production of a membrane bound form of said receptor is decreased.
34. The method of claim **32** or **33,** wherein said method is performed *in vivo.*
35. The method of claim **32** or **33,** wherein said method is performed *in vitro,*
36. The method of any one of claims **32-35,** wherein said receptor is a mammalian receptor selected from the group consisting of TNFRSF1A, TNFRSF1B, TNFRSF3, TNFRSF5, TNFRSF8, and TNFRSF11A.
37. The method claim **36,** wherein said mammalian receptor is a human, a primate, or a murine receptor.
38. The method of claim **37,** wherein said receptor is TNFRSF1A or TNFRSF1B.
39. The method of claim **38,** wherein said receptor is a human TNFRSF1A or a human TNFRSF1B.
40. The method of claim **39,** wherein said receptor is a human TNFRSF1B.
41. The method of claim **37,** wherein said SSO comprises from at least 10 to at least 20 nucleotides which are complementary to a contiguous sequence from SEQ ID Nos: 1, 2, 3 or 4.
42. The method of claim **41,** wherein said nucleotides are complementary to one or more nucleotides from the exon encoded by nucleotides 51-164 of SEQ ID No: 1, 51-79 of SEQ ID No: 2, 51-127 of SEQ ID No: 3, or 51-85 of SEQ ID No: 4.
43. The method of claim **42,** wherein said complementary sequence is complementary to at least 2, 5, 8 or 10 nucleotides of one of said exons.
44. The method of claim **42,** wherein said nucleotides are complementary only to sequences selected from said exons.
45. The method of claim **41,** wherein said SSOs are complementary only to an intron encoded by nucleotides 1-50 of SEQ ID No: 1, 165-215 of SEQ ID No: 1, 1-50 of SEQ ID No: 2, 80-130 of SEQ ID No: 2, 1-50 of SEQ ID No: 3, 128-178 of SEQ ID No: 3, nucleotides 1-50 of SEQ ID No: 4, or 86-136 of SEQ ID No: 4.
46. The method of any one of claims **32-45,** wherein said SSOs comprise one or more nucleotides or nucleosides independently selected from the group consisting of 2'-deoxyribonucleotides, 2'O-Me ribonucleotides, 2'O-MOE ribonucleotides, hexitol (HNA) nucleotides or nucleosides, 2'O-4'C-linked bicyclic ribofuranosyl (LNA) nucleotides or nucleosides, phosphorothioate analogs of any of the foregoing, peptide nucleic acid (PNA) analogs of any of the foregoing; methylphosponate analogs, of any of the foregoing, peptide nucleic acid analogs of any of the foregoing, N3'→P5' phosphoramidate analogs of any of the foregoing, and phosphorodiamidate morpholino nucleotide analogs of any of the foregoing, and combinations thereof.
47. The method of claim **46,** wherein said SSOs comprise only nucleotides or nucleosides independently selected from the group consisting of 2'-deoxyribonucleotides, 2'O-Me ribonucleotides, 2'O-MOE ribonucleotides, hexitol (HNA) nucleotides or nucleosides, 2'O-4'C-linked bicyclic ribofuranosyl (LNA) nucleotides or nucleosides, phosphorothioate analogs of any of the foregoing, peptide nucleic acid (PNA) analogs of any of the foregoing; methylphosponate analogs of any of the foregoing, peptide nucleic acid analogs of any of the foregoing, N3'→P5' phosphoramidate analogs of any of the foregoing, and phosphorodiamidate morpholino nucleotide analogs of any of the foregoing, and combinations thereof.
48. The method of claim **46,** wherein said 2'O-4'C-linked bicyclic ribofuranosyl (LNA) nucleotides or nucleosides are 2'O-4'C-(methylene)-ribofuranosyl nucleotides or nucleosides, respectively, or 2'O-4'C-(ethylene)-ribofuranosyl nucleotides or nucleosides, respectively.
49. The method of claim **46,** wherein said SSOs comprise one or more nucleotides or nucleosides independently selected from the group consisting of 2'O-Me ribonucleotides and 2'O-4'C-linked bicyclic ribofuranosyl (LNA) nucleotides or nucleosides.
50. The method of claim **46,** wherein said SSOs comprises at least four LNA nucleotides or nucleosides.
51. A splice switching oligomer (SSO) comprising from at least 10 to at least 20 nucleotides, said SSO capable of altering the splicing of a pre-mRNA encoding a receptor from the TNFR superfamily to produce a stable, secreted, ligand-binding form of said receptor.
52. The SSO of claim **51,** wherein said altering the splicing of said pre-mRNA comprises excising one or more exons encoding at least part of the membrane spanning domain of said receptor.
53. The SSO of claim **51-52,** wherein said receptor is a mammalian receptor selected from the group consisting of TNFRSF1A, TNFRSF1B, TNFRSF3, TNFRSF5, TNFRSF8, and TNFRSF11A.
54. The SSO of claim **53,** wherein said mammalian receptor is a human, a primate, or a murine receptor.
55. The SSO of claim **54,** wherein said receptor is TNFSF1A or TNFRSF1B.
56. The SSO of claim **55,** wherein said receptor is a human TNFSF1A or human TNFRSF1B.
57. The SSO of claim **56,** wherein said receptor is a human TNFSF1B.
58. The SSO of claim **56,** which comprises from at least 10 to at least 20 nucleotides which are complementary to a contiguous sequence from SEQ ID Nos: 1, 2, 3 or 4.
59. The SSO of claim **58** wherein said nucleotides are complementary to one or more nucleotides from the exon encoded by nucleotides 51-164 of SEQ ID No: 1, 51-79 of SEQ ID No: 2, 51-127 of SEQ ID No: 3, or 51-85 of SEQ ID No: 4.
60. The SSO of claim **59,** wherein said nucleotides are complementary to at least 2, 5, 8 or 10 nucleotides of one of said exons.
61. The SSO of claim **59,** wherein said nucleotides are complementary only to sequences selected from said exons.
62. The SSO of claim **58,** wherein said SSOs are complementary only to an intron encoded by nucleotides 1-50 of SEQ ID No: 1, 165-215 of SEQ ID No: 1, 1-50 of SEQ ID No: 2, 80-130 of SEQ ID No: 2, 1-50 of SEQ ID No: 3, 128-178 of SEQ ID No: 3, nucleotides 1-50 of SEQ ID No: 4, or 86-136 of SEQ ID No: 4.
63. The SSO of any one of claims **51-62,** wherein said SSO comprises one or more nucleotides or nucleosides independently selected from the group consisting of 2'-deoxyribonucleotides, 2'O-Me ribonucleotides, 2'O-MOE ribonucleotides, hexitol (HNA) nucleotides or nucleosides, 2'O-4'C-linked bicyclic ribofuranosyl (LNA) nucleotides or nucleosides, phosphorothioate analogs of any of the foregoing, peptide nucleic acid (PNA) analogs of any of the foregoing; methylphosponate analogs of any of the foregoing, peptide nucleic acid analogs of any of the foregoing, N3→P5' phosphoramidate analogs of any of the foregoing, and phosphorodiamidate morpholino nucleotide analogs of any of the foregoing, and combinations thereof.
64. The SSO of claim **63,** wherein said SSO comprises only nucleotides or nucleosides independently selected from the group consisting of 2'-deoxyribonucleotides, 2'O-Me ribonucleotides, 2'O-MOE ribonucleotides, hexitol (HNA) nucleotides or nucleosides, 2'O-4'C-linked bicyclic ribofuranosyl (LNA) nucleotides or nucleosides, phosphorothioate analogs of any of the foregoing, peptide nucleic acid (PNA) analogs of any of the foregoing; methylphosponate analogs of any of the foregoing, peptide nucleic acid analogs of any of the foregoing, N3'→P5' phosphoramidate analogs of any of the foregoing, and phosphorodimidate morpholino nucleotide analogs of any of the foregoing, and combinations thereof.
65. The SSO of claim 63, wherein said 2'O-4'C-linked bicyclic ribofuranosyl (LNA) nucleotides or nucleosides are 2'O-4'C-(methylene)-ribofurosyl nucleotides or nucleosides, respectively, or 2'O-4'C-(ethylene)-ribofuranosyl nucleotides or nucleosides, respectively.
66. The SSO of claim **63,** wherein said SSOs comprise one or more nucleotides or nucleosides independently selected from the group consisting of 2'O-Me ribonucleotides and 2'O-4'C-linked bicyclic ribofuranosyl (LNA) nucleotides or nucleosides.
67. The SSO of claim **63,** which contains at least four 2'O-4'-(methylene)-ribonucleoside phosphorothioates or 2'O-4'-(methylene)-ribonucleotides.
68. An SSO comprising a sequence selected from the group consisting of SEQ ID Nos: 8, 9, 14,17-21, 24-29, 32, 33, 38-42, 44-46, 50-52, 55-57, 60, 68-71, 74, 75, 77, 78, 80, 82, 84, and 86-89.
69. The SSO of claim **68,** wherein the sequence is complementary to a human TNFR2 sequence and is selected from the group consisting of SEQ ID Nos: 74, 75, 77, 78, 80, 82, and 84.
70. The SSO of claim **68,** wherein the sequence is complementary to a human TNFR2 sequence and is selected from the group consisting of SEQ ID Nos: 86-89.
71. The SSO of claim **69,** wherein the sequence is selected from the group consisting of SEQ ID Nos: 74, and 80.
72. A pharmaceutical composition comprising the SSO of any one of claims **51-71** and a pharmaceutically acceptable carrier.

## Claims

1. A splice switching oligomer of between 10 to 30 bases in length, which comprises a sequence that is complementary to at least 10 nucleotides of a portion of the human TNFR2 or TNFR1 genes, wherein said portion comprises either exon 7 and adjacent introns or exon 8 and adjacent introns.

2. The oligomer according to claim 1, which comprises a sequence that is complementary to at least 10 nucleotides of a sequence selected from SEQ ID NO 3, SEQ IN NO 4, SEQ ID NO 1, and SEQ ID NO 2.

3. The oligomer according to claim 1, which comprises a sequence that is complementary to at least 10 nucleotides of the portion of the human TNFR2 gene comprising exon 7 and the adjacent introns, such as SEQ ID NO 3.

4. The oligomer according to any one of claims 1 - 3 which comprises a sequence that is complementary to between 15 - 20 nucleotides of the respective portion of the human TNFR2 or TNFR1 genes.

5. The oligomer according to any one of claims 1 - 4, which has a length of between 12 to 24 nucleotides, such as a length of between 10 to 20 nucleotides.

6. The oligomer according to any one of claims 1 - 5, wherein said splice switching oligomer hybridises to a target RNA without activating the destruction of the target RNA by RNAseH.

7. The oligomer according to any one of claims 1 - 6, wherein said splice switching oligomer is capable of modulating the splicing of the pre-mRNA encoding the TNFR2 or TNFR1 receptor to induce the production and secretion of soluble TNFR2 (TNFRSF1 B) or soluble TNFR1 (TNFRSF1A) receptor.

8. The oligomer according to any one of claims 1 - 7 which comprise LNA nucleotides, such as oligomers which comprises both LNA nucleotides and non-LNA nucleotides.

9. The oligomer according to claim 8, wherein the oligomer has an alternating sequence of LNA and 2'deoxynucleotides.

10. The oligomer according to claim 9 which has a length of 16 nucleotides.

11. The oligomer according to any one of claims 1 -10 wherein the linkages are phosphorothioate linkages.

12. The oligomer according to any one of claims 1-11 which has a sequence selected from SEQ ID NOs 74, 75, 77, 78, 80, 82.

13. The oligomer according to any one of claims 1-11 which has a sequence selected from SEQ ID NOs 86, 87, 88 or 89.

14. The oligomer according to any one of claims 1 -13 for use as a medicament, such as for use in the treatment or amelioration of an inflammatory disease of a condition associated with TNF-alpha activity.

15. The use of an oligomer according to any one of claims 1 -13 for the preparation of a medicament for the treatment or amelioration of an inflammatory disease of a condition associated with TNF-alpha activity.


SEQUENCE LISTING

<110> SAZANI, PETER L.
      KOLE, RYSZARD
      ORUM, HENRIK

<120> SPLICE SWITCHING OLIGOMERS FOR TNF SUPERFAMILY RECEPTORS AND THEIR
      USE IN TREATMENT OF DISEASE

<130> 7353-4

<140>
<141>

<150> 60/862,350
<151> 2006-10-20

<150> 60/735,429
<151> 2005-11-10

<160> 116

<170> PatentIn version 3.3

<210> 1
<211> 214
<212> DNA
<213> Homo sapiens

<400> 1
tgcggccccc ctctgcccgc tcctctgacc aacacctgct ttgtctgcag gcaccacagt      60

gctgttgccc ctggtcattt tctttggtct ttgcctttta tccctcctct tcattggttt     120

aatgtatcgc taccaacggt ggaagtccaa gctctactcc attggtgagt gggggctttg     180

ggagggagag ggagctggtg ggggtgaggg agga                                 214


<210> 2
<211> 129
<212> DNA
<213> Homo sapiens

<400> 2
gggctgagag aggaagtgaa atttatgatg ctttctttct ttttcctcag tttgtgggaa      60

atcgacacct gaaaaagagg tgagatgaaa tgagagagtt actcccaaat gtccctgacc     120

attccttat                                                             129


<210> 3
<211> 178
<212> DNA
<213> Homo sapiens

<400> 3
acatttgagt ttgttttctg tagctgtctg agcttctctt ttctttctag gactgattgt      60

gggtgtgaca gccttgggtc tactaataat aggagtggtg aactgtgtca tcatgaccca     120

ggtgaaaagt aagagtccat ccttccttcc ttcatccact tgttcaggaa gcttttgt       178


<210> 4
<211> 135
<212> DNA
<213> Homo sapiens

<400> 4
gatgtgcctg aggaagtcaa tctcttactt gtcccctctc ctctttatag agaagccctt      60

gtgcctgcag agagaagcca aggtggtgag tgtctccact gccctctccc cctcttcccc     120

tggtctcctt cccgg                                                      135


<210> 5
<211> 20
<212> RNA
<213> Artificial Sequence

<220>
<223> Description of Artificial Sequence: Synthetic
      oligonucleotide

<400> 5
ccgcaguacc ugcagaccag                                                  20


<210> 6
<211> 20
<212> RNA
<213> Artificial Sequence

<220>
<223> Description of Artificial Sequence: Synthetic
      oligonucleotide

<400> 6
guaccugcag accagagagg                                                  20


<210> 7
<211> 20
<212> RNA
<213> Artificial Sequence

<220>
<223> Description of Artificial Sequence: Synthetic
      oligonucleotide

<400> 7
cugcagacca gagagguugc                                                  20


<210> 8
<211> 20
<212> RNA
<213> Artificial Sequence

<220>
<223> Description of Artificial Sequence: Synthetic
      oligonucleotide

<400> 8
acugauggag uagacuucgg                                                  20


<210> 9
<211> 20
<212> RNA
<213> Artificial Sequence

<220>
<223> Description of Artificial Sequence: Synthetic
      oligonucleotide

<400> 9

aguccuacuu acugauggag                                                20

<210> 10
<211> 20
<212> RNA
<213> Artificial Sequence

<220>
<223> Description of Artificial Sequence: Synthetic
      oligonucleotide

<400> 10
ccaaaguccu acuuacugau                                                20

<210> 11
<211> 20
<212> RNA
<213> Artificial Sequence

<220>
<223> Description of Artificial Sequence: Synthetic
      oligonucleotide

<400> 11
agauaaccag gggcaacagc                                                20

<210> 12
<211> 20
<212> RNA
<213> Artificial Sequence

<220>
<223> Description of Artificial Sequence: Synthetic
      oligonucleotide

<400> 12
aggauagaag gcaaagaccu                                                20

<210> 13
<211> 20
<212> RNA
<213> Artificial Sequence

<220>
<223> Description of Artificial Sequence: Synthetic
      oligonucleotide

<400> 13
ggcacauuaa acugaugaag                                                20

<210> 14
<211> 20
<212> RNA
<213> Artificial Sequence

<220>
<223> Description of Artificial Sequence: Synthetic
      oligonucleotide

<400> 14
ggccuccacc ggggauaucg                                                20

<210> 15
<211> 20

<212> RNA
<213> Artificial Sequence

<220>
<223> Description of Artificial Sequence: Synthetic
      oligonucleotide

<400> 15
cuggagaaca aagaaacaag                                                    20


<210> 16
<211> 20
<212> RNA
<213> Artificial Sequence

<220>
<223> Description of Artificial Sequence: Synthetic
      oligonucleotide

<400> 16
aucccuacaa acuggagaac                                                    20


<210> 17
<211> 20
<212> RNA
<213> Artificial Sequence

<220>
<223> Description of Artificial Sequence: Synthetic
      oligonucleotide

<400> 17
ggcacgggau cccuacaaac                                                    20


<210> 18
<211> 20
<212> RNA
<213> Artificial Sequence

<220>
<223> Description of Artificial Sequence: Synthetic
      oligonucleotide

<400> 18
cuucucaccu cuuugacagg                                                    20


<210> 19
<211> 20
<212> RNA
<213> Artificial Sequence

<220>
<223> Description of Artificial Sequence: Synthetic
      oligonucleotide

<400> 19
uggagucguc ccuucucacc                                                    20


<210> 20
<211> 20
<212> RNA
<213> Artificial Sequence

<220>
<223> Description of Artificial Sequence: Synthetic

oligonucleotide

<400> 20
cuccaacaau cagaccuagg                                                    20


<210> 21
<211> 20
<212> RNA
<213> Artificial Sequence

<220>
<223> Description of Artificial Sequence: Synthetic
      oligonucleotide

<400> 21
caaucagacc uaggaaaacg                                                    20


<210> 22
<211> 20
<212> RNA
<213> Artificial Sequence

<220>
<223> Description of Artificial Sequence: Synthetic
      oligonucleotide

<400> 22
agaccuagga aaacggcagg                                                    20


<210> 23
<211> 20
<212> RNA
<213> Artificial Sequence

<220>
<223> Description of Artificial Sequence: Synthetic
      oligonucleotide

<400> 23
ccuuacuuuu ccucugcacc                                                    20


<210> 24
<211> 20
<212> RNA
<213> Artificial Sequence

<220>
<223> Description of Artificial Sequence: Synthetic
      oligonucleotide

<400> 24
gagcagaacc uuacuuuucc                                                    20


<210> 25
<211> 20
<212> RNA
<213> Artificial Sequence

<220>
<223> Description of Artificial Sequence: Synthetic
      oligonucleotide

<400> 25
gacgagagca gaaccuuacu                                                    20

<210> 26
<211> 20
<212> RNA
<213> Artificial Sequence

<220>
<223> Description of Artificial Sequence: Synthetic
      oligonucleotide

<400> 26
ucagcagacc cagugauguc                                                   20


<210> 27
<211> 20
<212> RNA
<213> Artificial Sequence

<220>
<223> Description of Artificial Sequence: Synthetic
      oligonucleotide

<400> 27
augaugcagu ucaccagucc                                                   20


<210> 28
<211> 20
<212> RNA
<213> Artificial Sequence

<220>
<223> Description of Artificial Sequence: Synthetic
      oligonucleotide

<400> 28
ucaccagucc uaacaucagc                                                   20


<210> 29
<211> 20
<212> RNA
<213> Artificial Sequence

<220>
<223> Description of Artificial Sequence: Synthetic
      oligonucleotide

<400> 29
ccucugcacc aggaugaugc                                                   20


<210> 30
<211> 20
<212> RNA
<213> Artificial Sequence

<220>
<223> Description of Artificial Sequence: Synthetic
      oligonucleotide

<400> 30
uucucuacaa ugaagagagg                                                   20


<210> 31
<211> 20
<212> RNA
<213> Artificial Sequence

<220>
<223> Description of Artificial Sequence: Synthetic
      oligonucleotide

<400> 31
ggcuucucua caaugaagag                                                    20


<210> 32
<211> 20
<212> RNA
<213> Artificial Sequence

<220>
<223> Description of Artificial Sequence: Synthetic
      oligonucleotide

<400> 32
uguaggcagg agggcuucuc                                                    20


<210> 33
<211> 20
<212> RNA
<213> Artificial Sequence

<220>
<223> Description of Artificial Sequence: Synthetic
      oligonucleotide

<400> 33
acucaccacc uuggcaucuc                                                    20


<210> 34
<211> 20
<212> RNA
<213> Artificial Sequence

<220>
<223> Description of Artificial Sequence: Synthetic
      oligonucleotide

<400> 34
gcagagggau acucaccacc                                                    20


<210> 35
<211> 16
<212> DNA
<213> Artificial Sequence

<220>
<223> Description of Artificial Sequence: Synthetic
      oligonucleotide

<400> 35
caatcagacc taggaa                                                        16


<210> 36
<211> 16
<212> DNA
<213> Artificial Sequence

<220>
<223> Description of Artificial Sequence: Synthetic
      oligonucleotide

<400> 36
caacaatcag acctag                                                        16


<210> 37
<211> 16
<212> DNA
<213> Artificial Sequence

<220>
<223> Description of Artificial Sequence: Synthetic
      oligonucleotide

<400> 37
cagacctagg aaaacg                                                        16


<210> 38
<211> 16
<212> DNA
<213> Artificial Sequence

<220>
<223> Description of Artificial Sequence: Synthetic
      oligonucleotide

<400> 38
agcagaccca gtgatg                                                        16


<210> 39
<211> 16
<212> DNA
<213> Artificial Sequence

<220>
<223> Description of Artificial Sequence: Synthetic
      oligonucleotide

<400> 39
ccagtcctaa catcag                                                        16


<210> 40
<211> 16
<212> DNA
<213> Artificial Sequence

<220>
<223> Description of Artificial Sequence: Synthetic
      oligonucleotide

<400> 40
caccagtcct aacatc                                                        16


<210> 41
<211> 16
<212> DNA
<213> Artificial Sequence

<220>
<223> Description of Artificial Sequence: Synthetic
      oligonucleotide

<400> 41
ctgcaccagg atgatg                                                        16


<210> 42

<211> 16
<212> DNA
<213> Artificial Sequence

<220>
<223> Description of Artificial Sequence: Synthetic
      oligonucleotide

<400> 42
acttttcctc tgcacc                                                        16


<210> 43
<211> 16
<212> DNA
<213> Artificial Sequence

<220>
<223> Description of Artificial Sequence: Synthetic
      oligonucleotide

<400> 43
ccttactttt cctctg                                                        16


<210> 44
<211> 16
<212> DNA
<213> Artificial Sequence

<220>
<223> Description of Artificial Sequence: Synthetic
      oligonucleotide

<400> 44
cagaacctta cttttc                                                        16


<210> 45
<211> 16
<212> DNA
<213> Artificial Sequence

<220>
<223> Description of Artificial Sequence: Synthetic
      oligonucleotide

<400> 45
agagcagaac cttact                                                        16


<210> 46
<211> 16
<212> DNA
<213> Artificial Sequence

<220>
<223> Description of Artificial Sequence: Synthetic
      oligonucleotide

<400> 46
gagagcagaa ccttac                                                        16


<210> 47
<211> 16
<212> DNA
<213> Artificial Sequence

<220>

<223> Description of Artificial Sequence: Synthetic
      oligonucleotide

<400> 47
accttacttt tcctct                                                        16


<210> 48
<211> 16
<212> DNA
<213> Artificial Sequence

<220>
<223> Description of Artificial Sequence: Synthetic
      oligonucleotide

<400> 48
cttctctaca atgaag                                                        16


<210> 49
<211> 16
<212> DNA
<213> Artificial Sequence

<220>
<223> Description of Artificial Sequence: Synthetic
      oligonucleotide

<400> 49
ccttggcatc tctttg                                                        16


<210> 50
<211> 16
<212> DNA
<213> Artificial Sequence

<220>
<223> Description of Artificial Sequence: Synthetic
      oligonucleotide

<400> 50
tcaccacctt ggcatc                                                        16


<210> 51
<211> 16
<212> DNA
<213> Artificial Sequence

<220>
<223> Description of Artificial Sequence: Synthetic
      oligonucleotide

<400> 51
actcaccacc ttggca                                                        16


<210> 52
<211> 16
<212> DNA
<213> Artificial Sequence

<220>
<223> Description of Artificial Sequence: Synthetic
      oligonucleotide

<400> 52
gatactcacc accttg                                                        16

<210> 53
<211> 16
<212> DNA
<213> Artificial Sequence

<220>
<223> Description of Artificial Sequence: Synthetic
      oligonucleotide

<400> 53
ctacaatgaa gagagg                                                        16


<210> 54
<211> 16
<212> DNA
<213> Artificial Sequence

<220>
<223> Description of Artificial Sequence: Synthetic
      oligonucleotide

<400> 54
ctctacaatg aagaga                                                        16


<210> 55
<211> 16
<212> DNA
<213> Artificial Sequence

<220>
<223> Description of Artificial Sequence: Synthetic
      oligonucleotide

<400> 55
agggatactc accacc                                                        16


<210> 56
<211> 16
<212> DNA
<213> Artificial Sequence

<220>
<223> Description of Artificial Sequence: Synthetic
      oligonucleotide

<400> 56
cagagggata ctcacc                                                        16


<210> 57
<211> 16
<212> DNA
<213> Artificial Sequence

<220>
<223> Description of Artificial Sequence: Synthetic
      oligonucleotide

<400> 57
cgcagaggga tactca                                                        16


<210> 58
<211> 16
<212> DNA

<213> Artificial Sequence

<220>
<223> Description of Artificial Sequence: Synthetic
      oligonucleotide

<400> 58
gaacaagtca gaggca                                                        16


<210> 59
<211> 16
<212> DNA
<213> Artificial Sequence

<220>
<223> Description of Artificial Sequence: Synthetic
      oligonucleotide

<400> 59
gaggcaggac ttcttc                                                        16


<210> 60
<211> 16
<212> DNA
<213> Artificial Sequence

<220>
<223> Description of Artificial Sequence: Synthetic
      oligonucleotide

<400> 60
cgcagtacct gcagac                                                        16


<210> 61
<211> 16
<212> DNA
<213> Artificial Sequence

<220>
<223> Description of Artificial Sequence: Synthetic
      oligonucleotide

<400> 61
agtacctgca gaccag                                                        16


<210> 62
<211> 16
<212> DNA
<213> Artificial Sequence

<220>
<223> Description of Artificial Sequence: Synthetic
      oligonucleotide

<400> 62
ggcaacagca ccgcag                                                        16


<210> 63
<211> 16
<212> DNA
<213> Artificial Sequence

<220>
<223> Description of Artificial Sequence: Synthetic
      oligonucleotide

<400> 63
ctagcaagat aaccag                                                    16

<210> 64
<211> 16
<212> DNA
<213> Artificial Sequence

<220>
<223> Description of Artificial Sequence: Synthetic
      oligonucleotide

<400> 64
gcacattaaa ctgatg                                                    16

<210> 65
<211> 16
<212> DNA
<213> Artificial Sequence

<220>
<223> Description of Artificial Sequence: Synthetic
      oligonucleotide

<400> 65
cttcgggcct ccaccg                                                    16

<210> 66
<211> 16
<212> DNA
<213> Artificial Sequence

<220>
<223> Description of Artificial Sequence: Synthetic
      oligonucleotide

<400> 66
cttactgatg gagtag                                                    16

<210> 67
<211> 16
<212> DNA
<213> Artificial Sequence

<220>
<223> Description of Artificial Sequence: Synthetic
      oligonucleotide

<400> 67
cctacttact gatgga                                                    16

<210> 68
<211> 16
<212> DNA
<213> Artificial Sequence

<220>
<223> Description of Artificial Sequence: Synthetic
      oligonucleotide

<400> 68
gtcctactta ctgatg                                                    16

<210> 69
<211> 16
<212> DNA
<213> Artificial Sequence

<220>
<223> Description of Artificial Sequence: Synthetic
      oligonucleotide

<400> 69
tccctacaaa ctggag                                                    16


<210> 70
<211> 16
<212> DNA
<213> Artificial Sequence

<220>
<223> Description of Artificial Sequence: Synthetic
      oligonucleotide

<400> 70
ggcacgggat ccctac                                                    16


<210> 71
<211> 16
<212> DNA
<213> Artificial Sequence

<220>
<223> Description of Artificial Sequence: Synthetic
      oligonucleotide

<400> 71
ctctttgaca ggcacg                                                    16


<210> 72
<211> 16
<212> DNA
<213> Artificial Sequence

<220>
<223> Description of Artificial Sequence: Synthetic
      oligonucleotide

<400> 72
ctcacctctt tgacag                                                    16


<210> 73
<211> 16
<212> DNA
<213> Artificial Sequence

<220>
<223> Description of Artificial Sequence: Synthetic
      oligonucleotide

<400> 73
ccttctcacc tctttg                                                    16


<210> 74
<211> 16
<212> DNA
<213> Artificial Sequence

<220>
<223> Description of Artificial Sequence: Synthetic
      oligonucleotide

<400> 74
ccacaatcag tcctag                                                        16


<210> 75
<211> 16
<212> DNA
<213> Artificial Sequence

<220>
<223> Description of Artificial Sequence: Synthetic
      oligonucleotide

<400> 75
cagtcctaga aagaaa                                                        16


<210> 76
<211> 16
<212> DNA
<213> Artificial Sequence

<220>
<223> Description of Artificial Sequence: Synthetic
      oligonucleotide

<400> 76
agtagaccca aggctg                                                        16


<210> 77
<211> 16
<212> DNA
<213> Artificial Sequence

<220>
<223> Description of Artificial Sequence: Synthetic
      oligonucleotide

<400> 77
ccactcctat tattag                                                        16


<210> 78
<211> 16
<212> DNA
<213> Artificial Sequence

<220>
<223> Description of Artificial Sequence: Synthetic
      oligonucleotide

<400> 78
caccactcct attatt                                                        16


<210> 79
<211> 16
<212> DNA
<213> Artificial Sequence

<220>
<223> Description of Artificial Sequence: Synthetic
      oligonucleotide

<400> 79

ctgggtcatg atgaca                                                    16

<210> 80
<211> 16
<212> DNA
<213> Artificial Sequence

<220>
<223> Description of Artificial Sequence: Synthetic
      oligonucleotide

<400> 80
acttttcacc tgggtc                                                    16

<210> 81
<211> 16
<212> DNA
<213> Artificial Sequence

<220>
<223> Description of Artificial Sequence: Synthetic
      oligonucleotide

<400> 81
tcttactttt cacctg                                                    16

<210> 82
<211> 16
<212> DNA
<213> Artificial Sequence

<220>
<223> Description of Artificial Sequence: Synthetic
      oligonucleotide

<400> 82
tggactctta cttttc                                                    16

<210> 83
<211> 16
<212> DNA
<213> Artificial Sequence

<220>
<223> Description of Artificial Sequence: Synthetic
      oligonucleotide

<400> 83
aggatggact cttact                                                    16

<210> 84
<211> 16
<212> DNA
<213> Artificial Sequence

<220>
<223> Description of Artificial Sequence: Synthetic
      oligonucleotide

<400> 84
aaggatggac tcttac                                                    16

<210> 85
<211> 16

<212> DNA
<213> Artificial Sequence

<220>
<223> Description of Artificial Sequence: Synthetic
      oligonucleotide

<400> 85
cttctctata aagagg                                                        16


<210> 86
<211> 16
<212> DNA
<213> Artificial Sequence

<220>
<223> Description of Artificial Sequence: Synthetic
      oligonucleotide

<400> 86
ccttggcttc tctctg                                                        16


<210> 87
<211> 16
<212> DNA
<213> Artificial Sequence

<220>
<223> Description of Artificial Sequence: Synthetic
      oligonucleotide

<400> 87
tcaccacctt ggcttc                                                        16


<210> 88
<211> 16
<212> DNA
<213> Artificial Sequence

<220>
<223> Description of Artificial Sequence: Synthetic
      oligonucleotide

<400> 88
actcaccacc ttggct                                                        16


<210> 89
<211> 16
<212> DNA
<213> Artificial Sequence

<220>
<223> Description of Artificial Sequence: Synthetic
      oligonucleotide

<400> 89
gacactcacc accttg                                                        16


<210> 90
<211> 16
<212> DNA
<213> Artificial Sequence

<220>
<223> Description of Artificial Sequence: Synthetic

oligonucleotide

<400> 90
tgtggtgcct gcagac                                                        16


<210> 91
<211> 16
<212> DNA
<213> Artificial Sequence

<220>
<223> Description of Artificial Sequence: Synthetic
      oligonucleotide

<400> 91
ggtgcctgca gacaaa                                                        16


<210> 92
<211> 16
<212> DNA
<213> Artificial Sequence

<220>
<223> Description of Artificial Sequence: Synthetic
      oligonucleotide

<400> 92
ggcaacagca ctgtgg                                                        16


<210> 93
<211> 16
<212> DNA
<213> Artificial Sequence

<220>
<223> Description of Artificial Sequence: Synthetic
      oligonucleotide

<400> 93
caaagaaaat gaccag                                                        16


<210> 94
<211> 16
<212> DNA
<213> Artificial Sequence

<220>
<223> Description of Artificial Sequence: Synthetic
      oligonucleotide

<400> 94
atacattaaa ccaatg                                                        16


<210> 95
<211> 16
<212> DNA
<213> Artificial Sequence

<220>
<223> Description of Artificial Sequence: Synthetic
      oligonucleotide

<400> 95
gcttggactt ccaccg                                                        16

<210> 96
<211> 16
<212> DNA
<213> Artificial Sequence

<220>
<223> Description of Artificial Sequence: Synthetic
      oligonucleotide

<400> 96
ctcaccaatg gagtag                                                    16


<210> 97
<211> 16
<212> DNA
<213> Artificial Sequence

<220>
<223> Description of Artificial Sequence: Synthetic
      oligonucleotide

<400> 97
cactcaccaa tggagt                                                    16


<210> 98
<211> 16
<212> DNA
<213> Artificial Sequence

<220>
<223> Description of Artificial Sequence: Synthetic
      oligonucleotide

<400> 98
cccactcacc aatgga                                                    16


<210> 99
<211> 16
<212> DNA
<213> Artificial Sequence

<220>
<223> Description of Artificial Sequence: Synthetic
      oligonucleotide

<400> 99
cccccactca ccaatg                                                    16


<210> 100
<211> 16
<212> DNA
<213> Artificial Sequence

<220>
<223> Description of Artificial Sequence: Synthetic
      oligonucleotide

<400> 100
aaagccccca ctcacc                                                    16


<210> 101
<211> 16
<212> DNA
<213> Artificial Sequence

<220>
<223> Description of Artificial Sequence: Synthetic
      oligonucleotide

<400> 101
tttcccacaa actgag                                                    16


<210> 102
<211> 16
<212> DNA
<213> Artificial Sequence

<220>
<223> Description of Artificial Sequence: Synthetic
      oligonucleotide

<400> 102
ggtgtcgatt tcccac                                                    16


<210> 103
<211> 16
<212> DNA
<213> Artificial Sequence

<220>
<223> Description of Artificial Sequence: Synthetic
      oligonucleotide

<400> 103
ctctttttca ggtgtc                                                    16


<210> 104
<211> 16
<212> DNA
<213> Artificial Sequence

<220>
<223> Description of Artificial Sequence: Synthetic
      oligonucleotide

<400> 104
ctcacctctt tttcag                                                    16


<210> 105
<211> 16
<212> DNA
<213> Artificial Sequence

<220>
<223> Description of Artificial Sequence: Synthetic
      oligonucleotide

<400> 105
tcatctcacc tctttt                                                    16


<210> 106
<211> 16
<212> DNA
<213> Artificial Sequence

<220>
<223> Description of Artificial Sequence: Synthetic
      oligonucleotide

<400> 106
gctattacct taaccc                                                    16


<210> 107
<211> 214
<212> DNA
<213> Mus musculus

<400> 107
cccctagtct ctgctgtggc ctcacactga gcaacctctc tggtctgcag gtactgcggt      60

gctgttgccc ctggttatct tgctaggtct ttgccttcta tcctttatct tcatcagttt     120

aatgtgccga tatccccggt ggaggcccga agtctactcc atcagtaagt aggactttgg     180

ggatataggg tgttggtgga gatacgggag gggt                                 214


<210> 108
<211> 129
<212> DNA
<213> Mus musculus

<400> 108
gcgttgaaag ggaagtgaaa ttcatgacac cttgtttctt tgttctccag tttgtaggga      60

tcccgtgcct gtcaaagagg tgagaaggga cgactccagc ttccctgact actccttcca     120

acgcctgat                                                             129


<210> 109
<211> 178
<212> DNA
<213> Mus musculus

<400> 109
caccagccac cctggaacct ttgtttctga gtaccctgcc gttttcctag gtctgattgt      60

tggagtgaca tcactgggtc tgctgatgtt aggactggtg aactgcatca tcctggtgca     120

gaggaaaagt aaggttctgc tctcgtcctg tttcccgccc cacgtcccta ccctaaca       178


<210> 110
<211> 135
<212> DNA
<213> Mus musculus

<400> 110
ctgttctgaa gaagtcctgc ctctgacttg ttcccctctc ttcattgtag agaagccctc      60

ctgcctacaa agagatgcca aggtggtgag tatccctctg cggtcctcct cccccttctc      120

tcctccagct ctccc                                                      135


<210> 111
<211> 21
<212> DNA
<213> Artificial Sequence

<220>
<223> Description of Artificial Sequence: Synthetic
      oligonucleotide

<400> 111
gaaagtgagt gcgtcccttg c                                               21

<210> 112
<211> 20
<212> DNA
<213> Artificial Sequence

<220>
<223> Description of Artificial Sequence: Synthetic
      oligonucleotide

<400> 112
gcacggagca gagtgattcg                                                    20


<210> 113
<211> 21
<212> DNA
<213> Artificial Sequence

<220>
<223> Description of Artificial Sequence: Synthetic
      oligonucleotide

<400> 113
gagccccaaa tggaaatgtg c                                                  21


<210> 114
<211> 17
<212> DNA
<213> Artificial Sequence

<220>
<223> Description of Artificial Sequence: Synthetic
      oligonucleotide

<400> 114
gctcaaggcc tactgcc                                                       17


<210> 115
<211> 24
<212> DNA
<213> Artificial Sequence

<220>
<223> Description of Artificial Sequence: Synthetic
      oligonucleotide

<400> 115
actgaaacat cagacgtggt gtgc                                               24


<210> 116
<211> 21
<212> DNA
<213> Artificial Sequence

<220>
<223> Description of Artificial Sequence: Synthetic
      oligonucleotide

<400> 116
ccttatcggc aggcaagtga g                                                  21